# EUROPEAN PATENT SPECIFICATION

(11) **EP 3 233 008 B1**
(45) Date of publication and mention of the grant of the patent: **13.11.2019**
(21) Application number: 15817732.9
(22) Date of filing: 15.12.2015
(51) Int. Cl.: A61F 13/514, A61F 13/15

(54) **APPARATUSES AND METHODS FOR MAKING ABSORBENT ARTICLES WITH LOW INTENSITY INNER BELT EDGE AND LEG OPENING EDGE REGIONS**
VORRICHTUNGEN UND VERFAHREN ZUR HERSTELLUNG SAUGFÄHIGER ARTIKEL MIT INNENBANDRAND MIT NIEDRIGER INTENSITÄT UND BEINÖFFNUNGSRANDBEREICHEN
APPAREILS ET PROCÉDÉS DE FABRICATION D'ARTICLES ABSORBANTS AVEC DES RÉGIONS DE BORD DE CEINTURE INTÉRIEURE ET DE BORD D'OUVERTURE DE JAMBES DE FAIBLE DENSITÉ

(30) Priority: 18.12.2014 US 201462093452 P
(43) Date of publication of application: 25.10.2017
(73) Proprietor: The Procter & Gamble Company, Cincinnati, OH 45202 (US)
(72) Inventor: ZINK, Ronald, Joseph, II, Cincinnati, Ohio 45202 (US); SAUER, Linda, Ann, Cincinnati, Ohio 45202 (US)
(74) Representative: P&G Patent Germany
(86) International application number: PCT/US2015/065655
(87) International publication number: WO 2016/100246

(56) References cited:
- US-A1- 2013 255 861
- US-A1- 2013 255 862

## Description

### FIELD OF THE INVENTION

The present disclosure relates to methods for manufacturing absorbent articles, and more particularly, to assembling absorbent articles with components having graphics including zones of relatively high print densities and zones of relatively low print densities, wherein the zones of relatively low print densities are positioned in regions of assembled components that are subject to various process transformations during assembly.

### BACKGROUND OF THE INVENTION

Along an assembly line, diapers and various types of other disposable absorbent articles may be assembled by adding components to and otherwise modifying advancing, continuous webs of material. Webs of material and component parts used to manufacture diapers may include: backsheets, topsheets, absorbent cores, front and/or back ears, fastener components, and various types of elastic webs and components such as leg elastics, barrier leg cuff elastics, and waist elastics.

Some consumers may prefer purchasing absorbent articles, such as diapers, having various types of different graphic designs printed thereon. As such, continuous substrates of material having printed graphics may be converted into different components used to assemble the absorbent articles. During the assembly process, the substrates of material having the graphics printed thereon may be subjected to various process transformations, such as folding, bonding, trimming, and/or cutting.

In some instances, consumers may prefer diapers with graphics defining various designs and various colored areas that may be printed thereon and that may extend over the entire area, or a relatively large area, of the diaper that is visible when worn. Thus, in converting operations involving the assembly of diapers having printed graphics that extend over relatively large regions, the printed substrates may be subjected to various process transformations in areas where the printing is located. However, subjecting printed substrates to various process transformations, such as folding, cutting, bonding, and/or assemblage with other printed components in areas where the graphics are located may create challenges in performing such process transformations when attempting to maintain aesthetically pleasing final assemblies. For example, imprecise and/or inconsistent bonding, cutting, and/or folding operations performed on a substrate in an area where a printed graphic is located may act to visibly highlight such process imprecisions or inconsistencies, such as crooked bond lines, fold lines, and/or cut lines. In another example, imprecise placement of one printed component onto another printed component may be visibly highlighted when graphics on the separate components appear disjointed and/or misaligned when the components are combined. In addition, the aforementioned challenges may be exacerbated in absorbent article assembly processes operating at relatively high speed production rates.

Consequently, there remains a need to incorporate substrates and/or components into absorbent article assembly processes wherein the substrates and/or components include graphics printed and/or positioned in such a manner so as to functionally reduce noticeable visible results of imprecise and/or inconsistent manufacturing operations performed in areas where the graphics are located.

US 2013/255862A1 relates to methods and apparatuses for assembling diapers, each including a chassis connected with front and back elastic belts. Opposing end regions of the chassis are connected with regions of the elastic belts where the elasticity of the elastic belts has been removed or deactivated. An elastic laminate may be formed by continuously bonding elastic strands between a first continuous substrate layer and a second continuous substrate layer such that the elastic strands are bonded to both the first substrate layer and the second substrate layer in heavy-bond regions. And the elastic strands are bonded to the first substrate layer and/or the second substrate layer with in light-bond regions. The elastic strands are then intermittently deactivated by cutting the strands into one or more discrete pieces in the light-bond regions. The discrete pieces retract and remain in the light-bond regions.

US 2013/255861A1 relates to methods and apparatuses for assembling absorbent articles, each including a chassis connected with front and back elastic belts. Opposing end regions of the chassis are connected with regions of the elastic belts where the elasticity of the elastic belts has been removed or deactivated. An elastic laminate is formed by intermittently bonding elastic strands between a first continuous substrate layer and a second continuous substrate layer. The elastic strands are then intermittently deactivated by severing the strands in the non-bonded regions to form deactivated regions of the elastic laminate. A plurality of chassis may then be bonded with the elastic laminate, wherein the first or second end regions of each chassis may be bonded with deactivated regions of the continuous elastic laminate.

### SUMMARY OF THE INVENTION

The present disclosure relates to absorbent articles and methods for assembling absorbent articles with substrates and/or components that include graphics that may be positioned and/or printed in such a manner so as to reduce noticeable visible results of imprecise and/or inconsistent manufacturing operations performed in areas where the graphics are located.

In one form, in a method for assembling disposable diaper pants, each diaper pant comprising a chassis having a first end region and an opposing second end region separated from each other by a central region, and having a longitudinal axis and a lateral axis, the chassis comprising: a topsheet, a backsheet, and an absorbent core disposed between the topsheet and the backsheet, the method comprises the steps of: advancing a continuous elastic laminate in a machine direction, the elastic laminate comprising a first longitudinal edge and a second longitudinal edge defining a width, W, in a cross direction, the elastic laminate further comprising a graphic, the graphic extending in the machine direction and the cross direction and comprising a central zone positioned between laterally opposing first and second zones, wherein each zone comprises a maximum print density, wherein the maximum print density of the central zone is less than or equal to about 30% of the maximum print densities of the first and second zones, and wherein the central zone defines a width, Wz, in the cross direction of less than or equal to about 10% of the width, W, of the elastic laminate; cutting the elastic laminate along the machine direction and through the central zone to form a first continuous elastic laminate and a second continuous elastic laminate, wherein the first and second continuous elastic laminates each include an inner longitudinal edge and an outer longitudinal edge, and wherein a first portion of the central zone extends along inner longitudinal edge of the first continuous elastic laminate and a second portion of the central zone extends along the inner longitudinal edge of the second continuous elastic laminate; separating the first continuous elastic laminate in the cross direction from the second continuous elastic laminate to define a gap between the inner longitudinal edge of the first continuous elastic laminate and the inner longitudinal edge of the second continuous elastic laminate; depositing a plurality of chassis spaced apart from each other along the machine direction across the gap and onto the first continuous elastic laminate and the second continuous elastic laminate; folding each chassis along the lateral axis to position the first continuous elastic laminate into a facing relationship with the second continuous elastic laminate; and cutting the first and second continuous elastic laminates in the cross direction to form discrete diaper pants.

In another form, in a method for assembling disposable diaper pants, each diaper pant comprising a chassis having a first end region and an opposing second end region separated from each other by a central region, and having a longitudinal axis and a lateral axis, the chassis comprising: a topsheet, a backsheet, and an absorbent core disposed between the topsheet and the backsheet, the method comprises the steps of: advancing a first continuous elastic laminate in a machine direction comprising an outer longitudinal edge and an inner longitudinal edge defining a first width, W1, in a cross direction, the first continuous elastic laminate further comprising a first graphic, the first graphic extending in the machine direction and the cross direction and comprising a first zone and a second zone, wherein the second zone is positioned between the inner longitudinal edge and the first zone, wherein the first zone comprises a first maximum print density and the second zone comprises a second maximum print density, wherein the second maximum print density is less than or equal to about 30% of the first maximum print density, and wherein the second zone defines a width, Wz, in the cross direction that is less than or equal to about 10% of the first width, W1, of the first continuous substrate; advancing a second continuous elastic laminate comprising an outer longitudinal edge and an inner longitudinal edge; separating the first continuous elastic laminate in the cross direction from the second continuous elastic laminate to define a gap between the inner longitudinal edge of the first continuous elastic laminate and the inner longitudinal edge of the second continuous elastic laminate; depositing a plurality of chassis spaced apart from each other along the machine direction across the gap and onto the first continuous elastic laminate and the second continuous elastic laminate, wherein at least one chassis comprises a second graphic having a first zone and a second zone, wherein the first zone comprises a first maximum print density and the second zone comprises a second maximum print density, wherein the second maximum print density is less than or equal to about 25% of the first maximum print density; positioning the at least one chassis to align the second zone of the second graphic with the second zone of the first graphic to form a contiguous design; folding each chassis along the lateral axis to position the first continuous elastic laminate into a facing relationship with the second continuous elastic laminate; and cutting the first and second continuous elastic laminates in the cross direction to form discrete diaper pants.

In yet another form, in a method for assembling disposable diaper pants, each diaper pant comprising a chassis having a first end region and an opposing second end region separated from each other by a central region, and having a longitudinal axis and a lateral axis, the chassis comprising: a topsheet, a backsheet, and an absorbent core disposed between the topsheet and the backsheet, the method comprises the steps of: advancing a continuous elastic laminate in a machine direction, wherein the first continuous elastic laminate comprises a first substrate having a first surface and an opposing second surface, a second substrate having a first surface and an opposing second surface, and elastic material bonded between the first surfaces of the first and second substrates, the first substrate comprising a first longitudinal edge and a second longitudinal edge defining a width, W, in a cross direction, the elastic laminate further comprising a graphic, the graphic extending in the machine direction and the cross direction and comprising a central zone positioned between laterally opposing first and second zones, wherein each zone comprises a maximum print density, wherein the maximum print density of the central zone is less than or equal to about 30% of the maximum print densities of the first and second zones, and wherein the central zone defines a width, Wz, in the cross direction is less than or equal to about 10% of the width, W, of the elastic laminate; cutting holes in the first substrate, wherein the holes are spaced apart from each other along the machine direction and wherein perimeters of the holes extend through the central zone; depositing a plurality of chassis spaced apart from each other along the machine direction and onto the first substrate; folding each chassis along the lateral axis; and cutting the elastic laminate in the cross direction to form discrete diaper pants.

### BRIEF DESCRIPTION OF THE DRAWINGS

Figure 1A is a front perspective view of a diaper pant.
Figure 1B is a rear perspective view of a diaper pant.
Figure 2A is a partially cut away plan view of the diaper pant shown in Figures 1A and 1B in a flat, uncontracted state.
Figure 2B is a plan view of the diaper pant shown in Figures 1A and 1B in a flat, uncontracted state and including graphics with low intensity zones positioned along front and rear inner belt edges.
Figure 3A is a cross-sectional view of the diaper pant of Figure 2A taken along line 3A-3A.
Figure 3B is a cross-sectional view of the diaper pant of Figure 2A taken along line 3B-3B.
Figure 4 is a schematic side view of a converting apparatus adapted to manufacture pre-fastened, pant diapers.
Figure 5A1 is a view of a continuous length of an advancing first substrate from Figure 4 taken along line A1-A1.

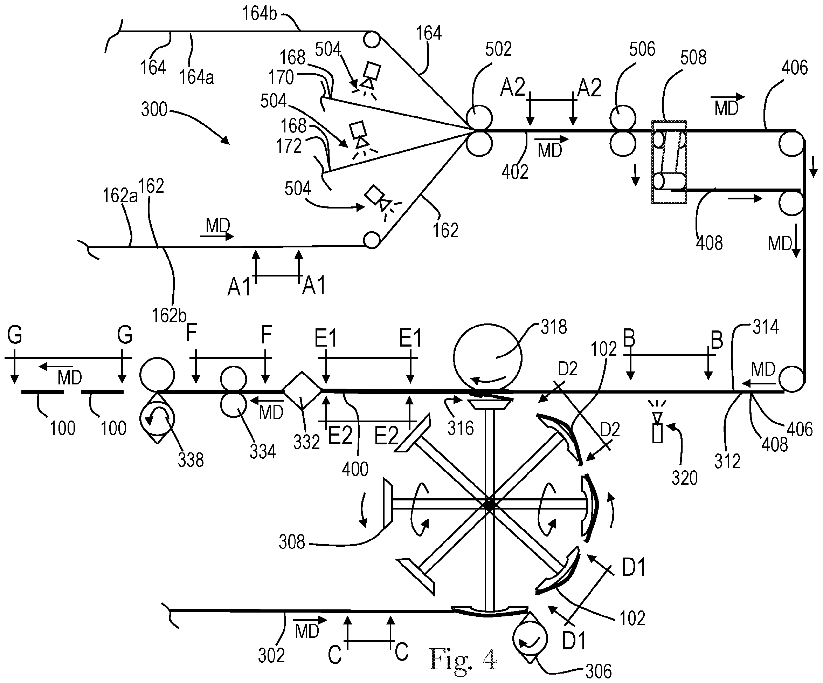

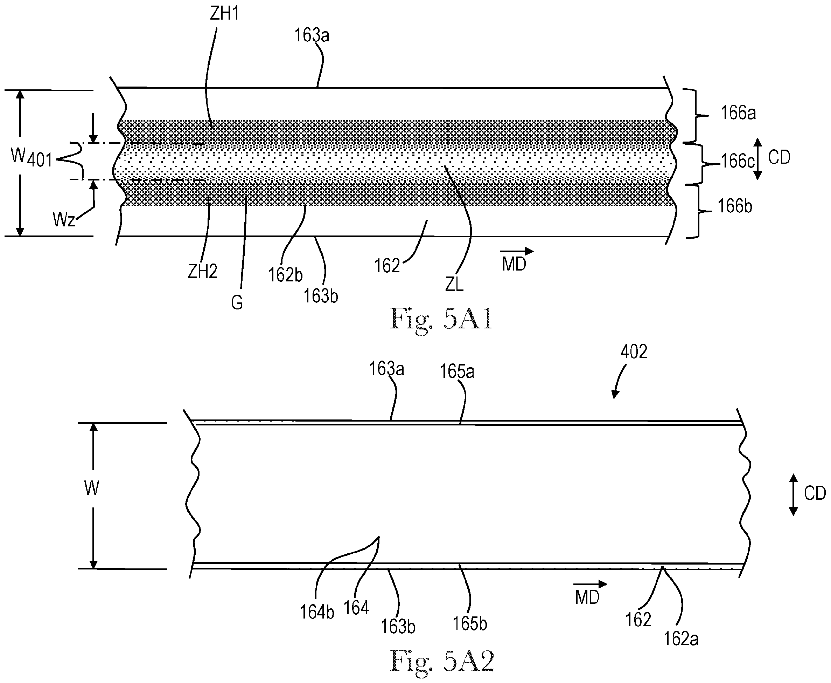

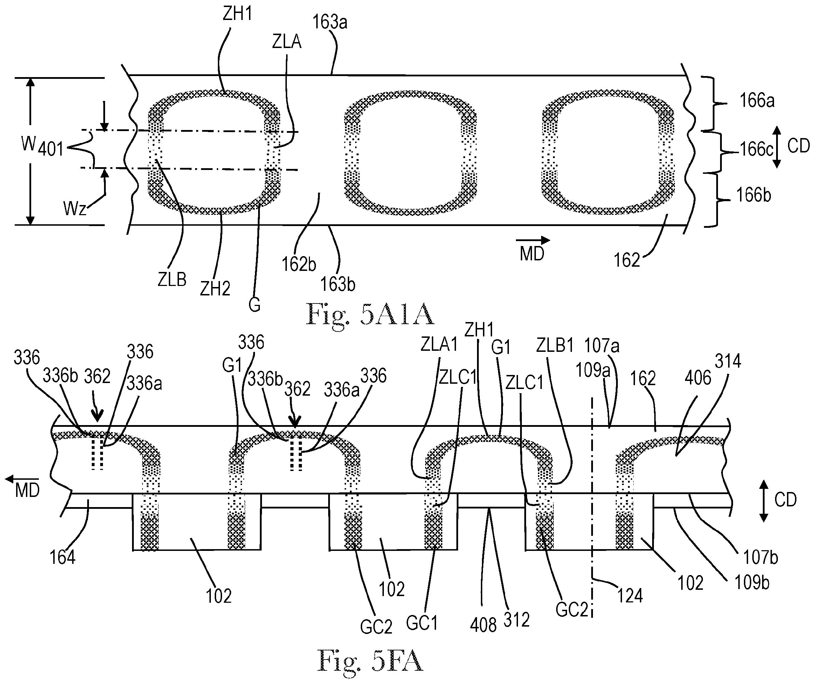

Figure 5A2 is a view of a continuous length of an advancing elastic laminate from Figure 4 taken along line A2-A2.

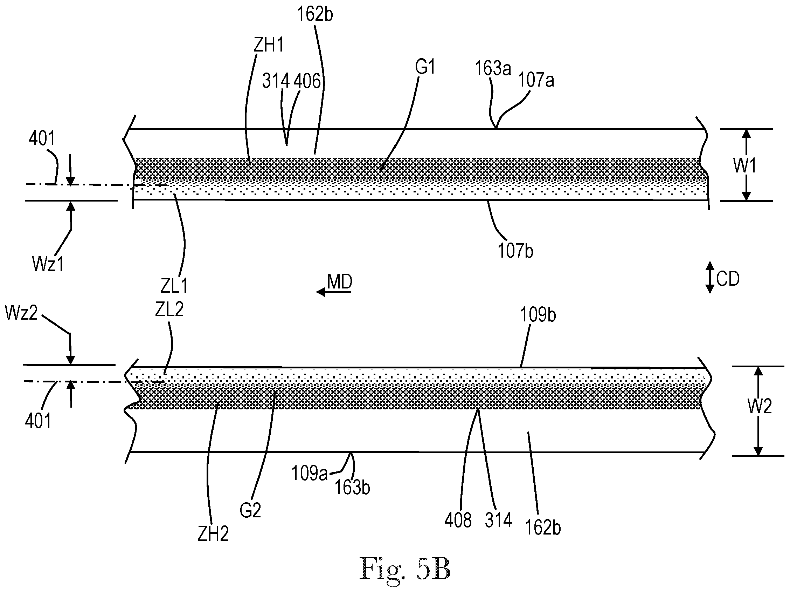

Figure 5B is a view of continuous lengths of advancing first and second elastic belt laminates from Figure 4 taken along line B-B.

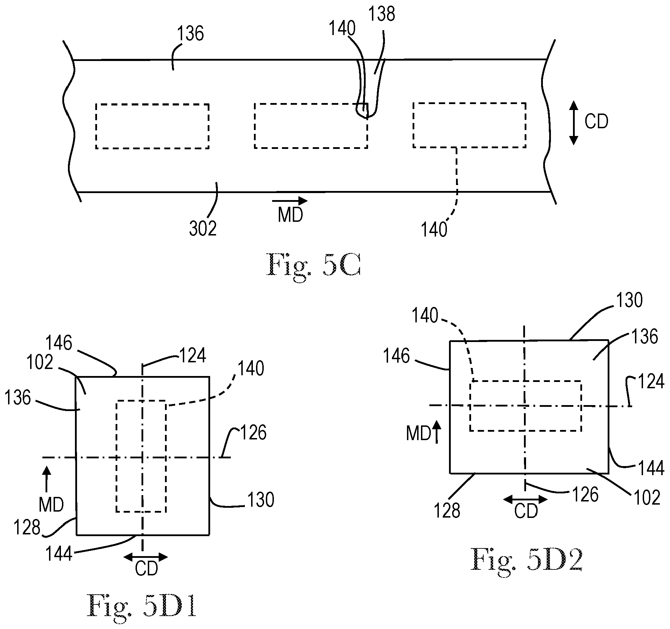

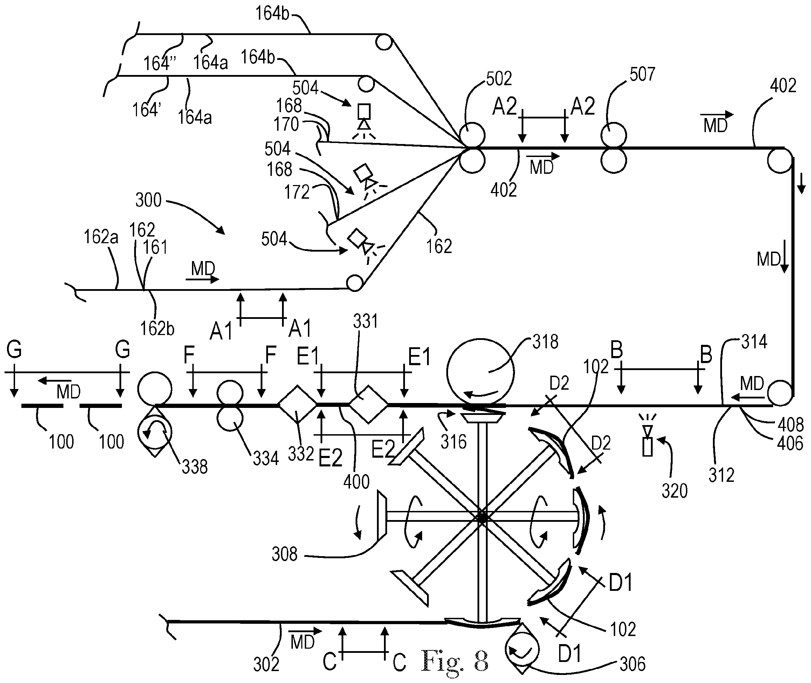

Figure 5C is a view of a continuous length of chassis assemblies from Figures 4 and 8 taken along line C-C.

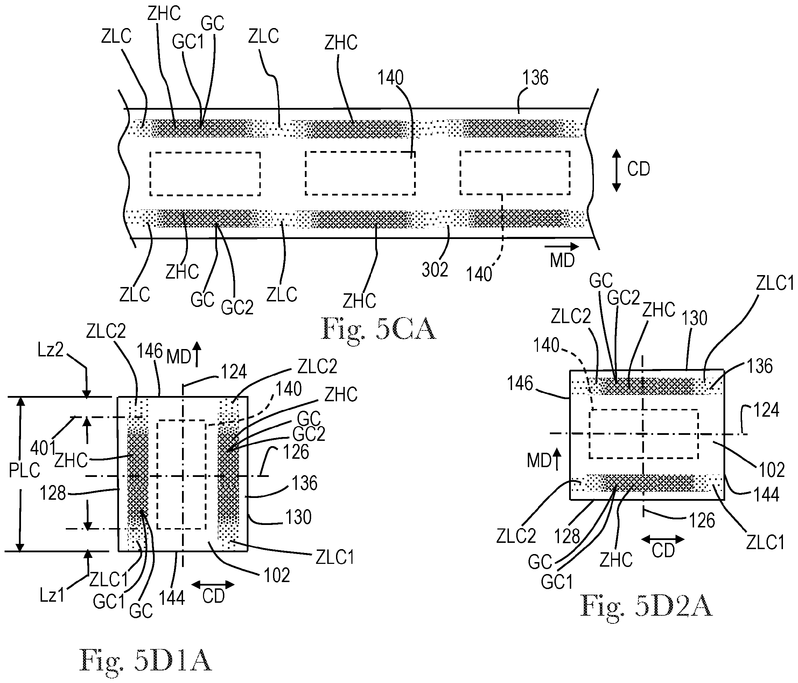

Figure 5D1 is a view of a discrete chassis from Figures 4 and 8 taken along line D1-D1.
Figure 5D2 is a view of a discrete chassis from Figures 4 and 8 taken along line D2-D2.

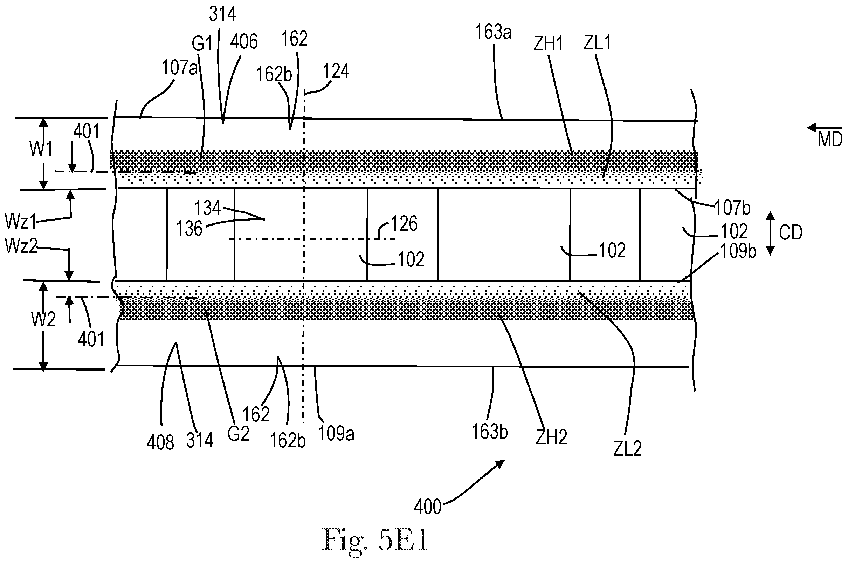

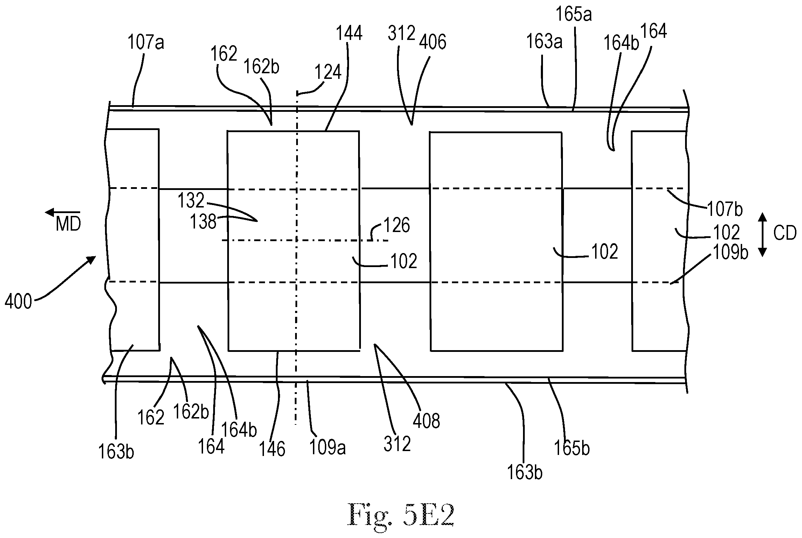

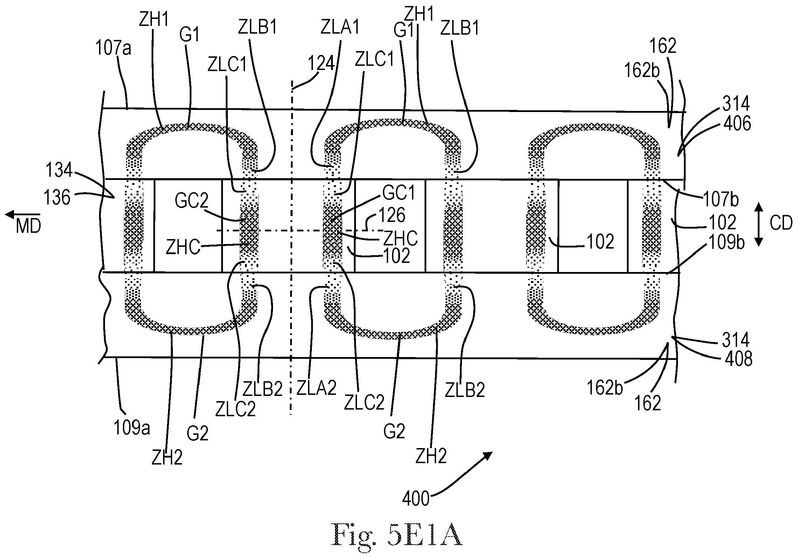

Figure 5E1 is a view of multiple discrete chassis spaced from each other along the machine direction MD and connected with each other by the first and second elastic belt laminates from Figure 4 taken along line E1-E1.
Figure 5E2 is a view of multiple discrete chassis spaced from each other along the machine direction MD and connected with each other by the first and second elastic belt laminates from Figure 4 taken along line E2-E2.

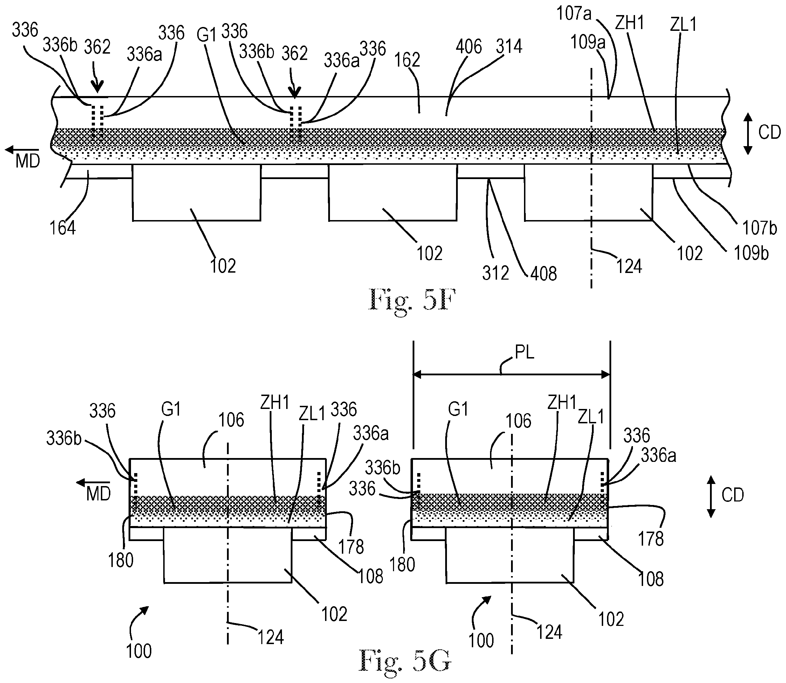

Figure 5F is a view of folded multiple discrete chassis with the first and second elastic belt laminates in a facing relationship from Figure 4 taken along line F-F.
Figure 5G is a view of two discrete absorbent articles advancing the machine direction MD from Figure 4 taken along line G-G.
Figure 5A1A is a view of a continuous length of an advancing first substrate from Figure 4 taken along line A1-A1 and showing a second embodiment of a graphic configuration.

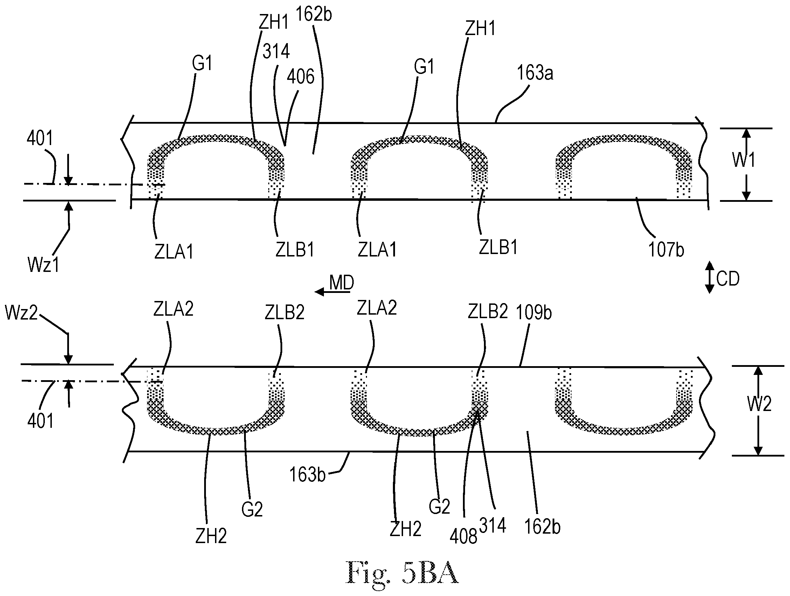

Figure 5BA is a view of continuous lengths of advancing first and second elastic belt laminates from Figure 4 taken along line B-B and showing a second embodiment of a graphic configuration.
Figure 5CA is a view of a continuous length of chassis assemblies with chassis graphics from Figure 4 taken along line C-C.
Figure 5D1A is a view of a discrete chassis with chassis graphics from Figure 4 taken along line D1-D1.
Figure 5D2A is a view of a discrete chassis from Figure 4 with chassis graphics taken along line D2-D2.
Figure 5E1A is a view of multiple discrete chassis spaced from each other along the machine direction MD and connected with each other by the first and second elastic belt laminates from Figure 4 taken along line E1-E1 and showing a second embodiment of a graphic configuration.
Figure 5FA is a view of folded multiple discrete chassis with chassis graphics and showing the first and second elastic belt laminates with a second embodiment of a graphic configuration in a facing relationship from Figure 4 taken along line F-F.

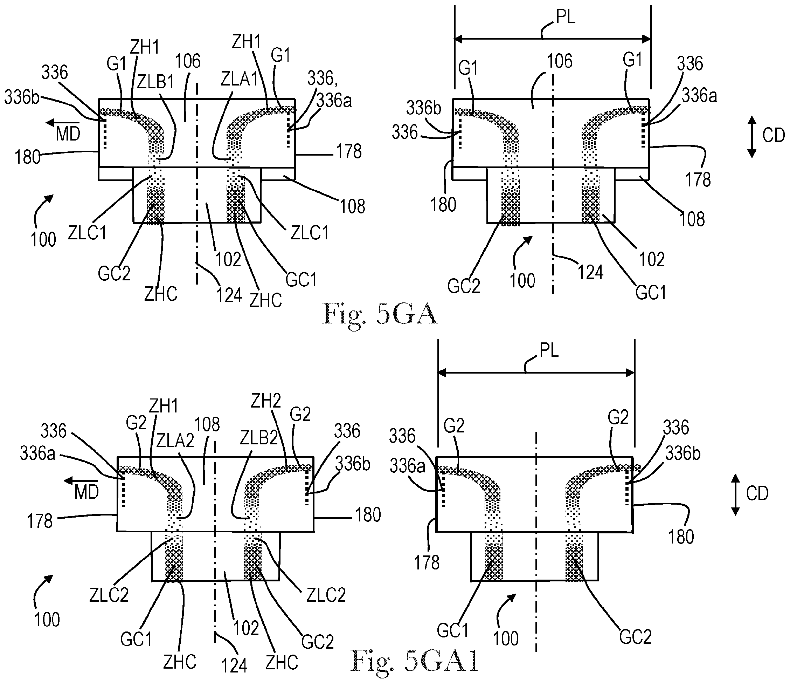

Figure 5GA is a front view of two discrete absorbent articles having chassis graphics and a second embodiment of a graphic configuration on front and rear elastic belts advancing the machine direction MD from Figure 4 taken along line G-G.
Figure 5GA1 is a rear view of the two discrete absorbent articles from Figure 5GA.

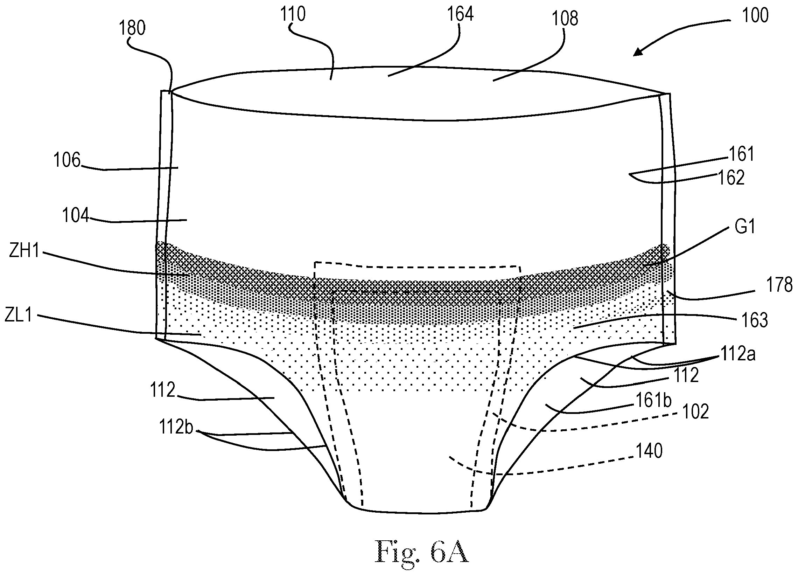

Figure 6A is a front perspective view of a diaper pant constructed with a contiguous outer cover.

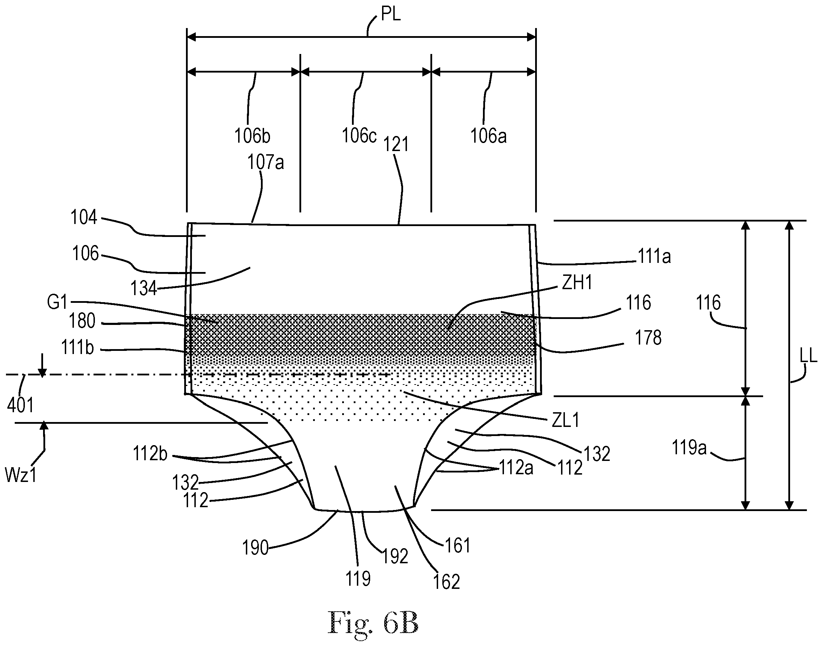

Figure 6B is a front plan view of the diaper pant of Figure 6A.

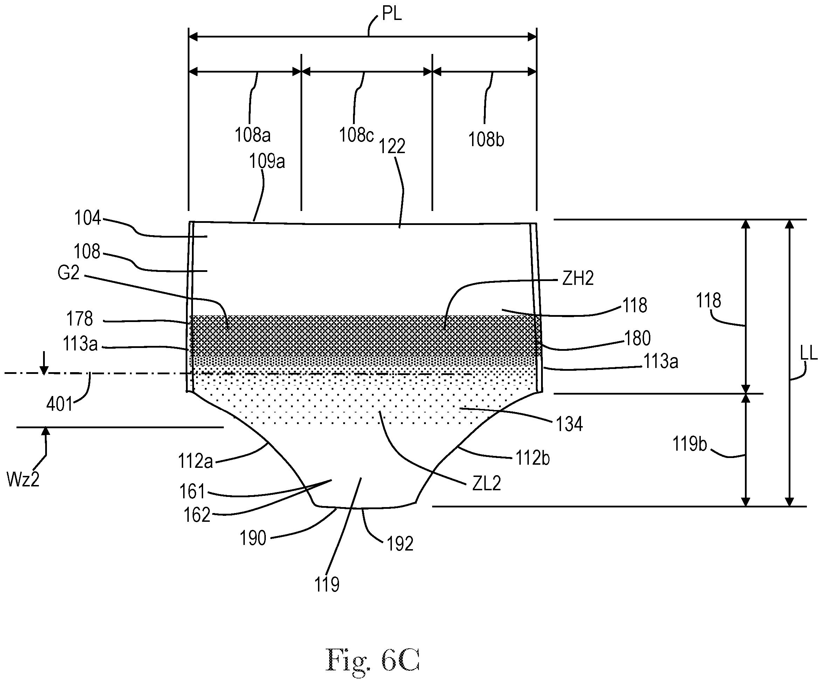

Figure 6C is a rear plan view of the diaper pant of Figure 6A.

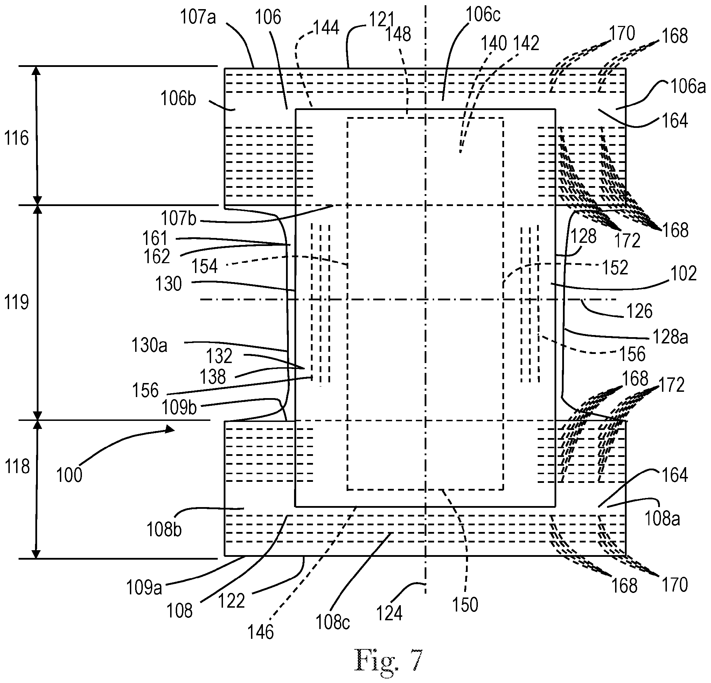

Figure 7 is a partially cut away plan view of the diaper pant shown in Figures 6A-6C in a flat, uncontracted state.
Figure 8 is a schematic side view of a converting apparatus adapted to manufacture pre-fastened, pant diapers.

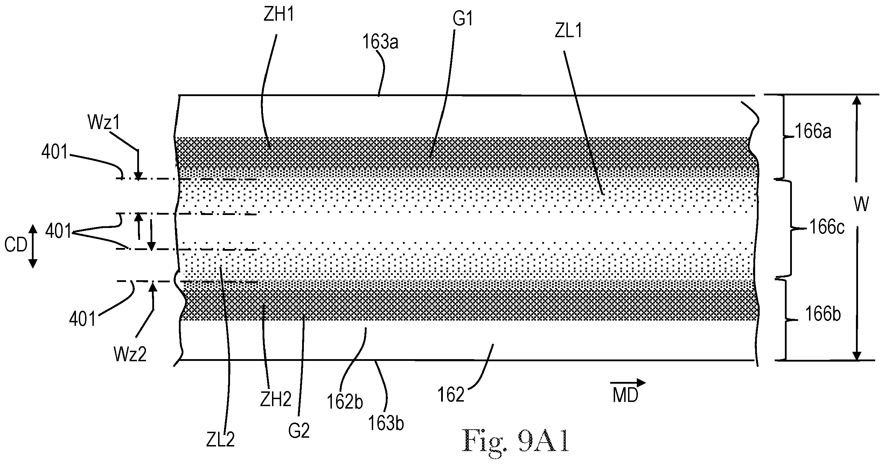

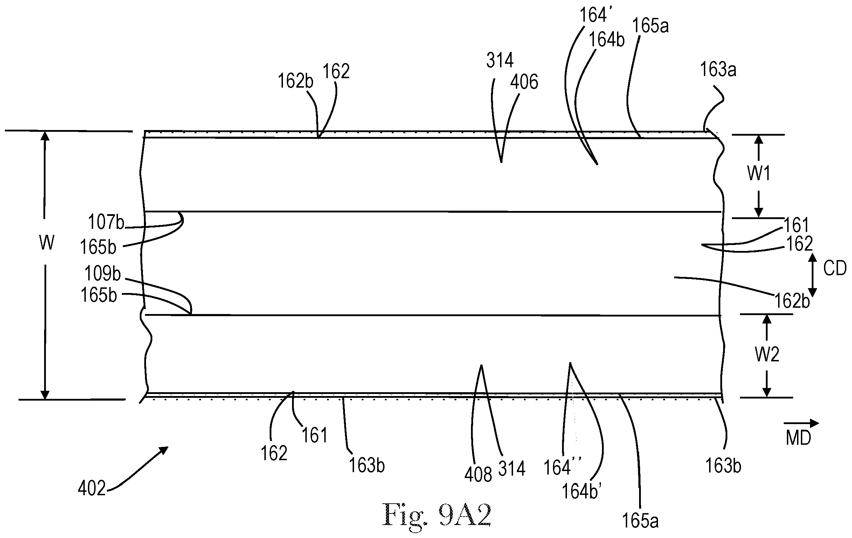

Figure 9A1 is a view of a continuous length of an advancing first substrate from Figure 8 taken along line A1-A1.
Figure 9A2 is a view of a continuous length of an advancing elastic laminate from Figure 8 taken along line A2-A2.

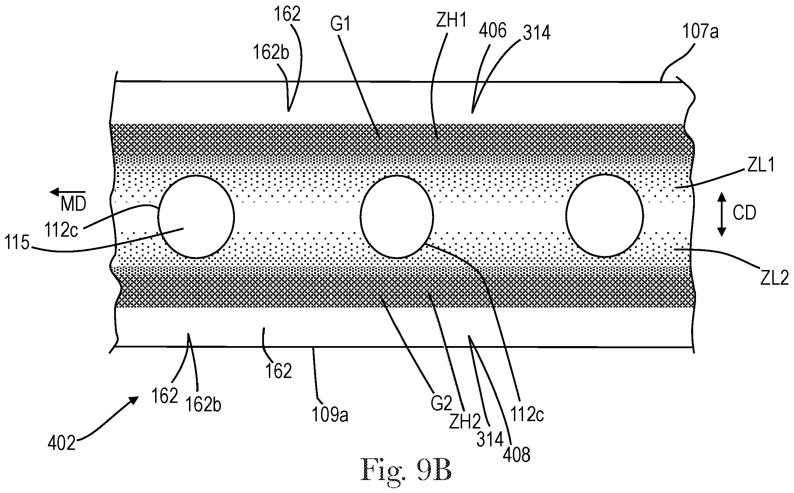

Figure 9B is a view of continuous lengths of advancing first and second elastic belt laminates from Figure 8 taken along line B-B.

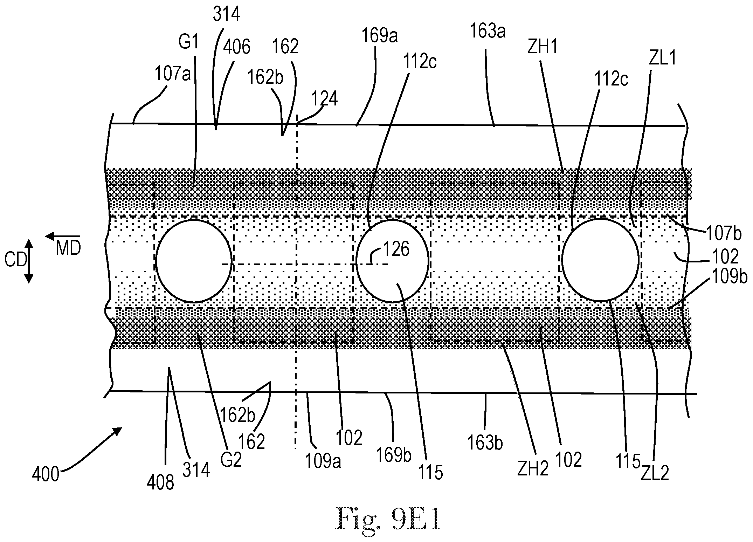

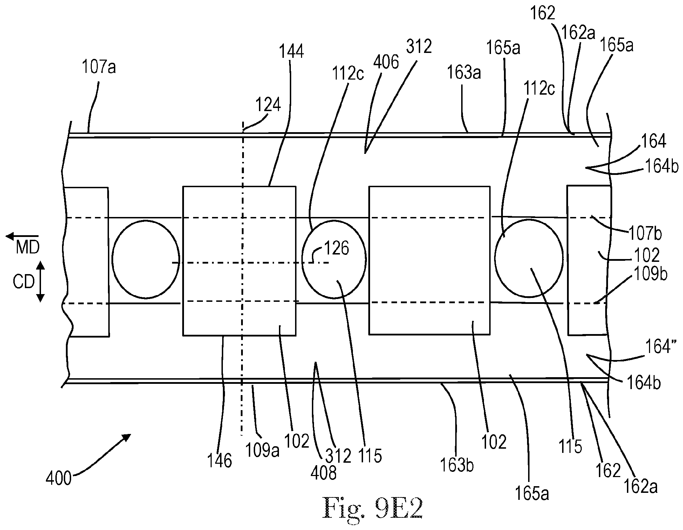

Figure 9E1 is a view of multiple discrete chassis spaced from each other along the machine direction MD and connected with each other by an outer cover and the first and second elastic belt laminates from Figure 8 taken along line E1-E1.
Figure 9E2 is a view of multiple discrete chassis spaced from each other along the machine direction MD and connected with each other by an outer cover and the first and second elastic belt laminates from Figure 8 taken along line E2-E2.

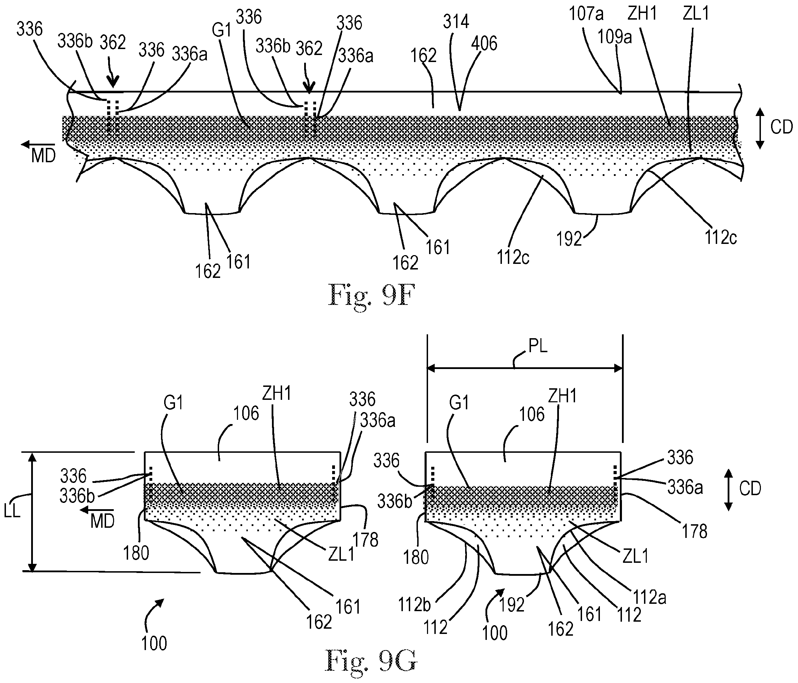

Figure 9F is a view of folded multiple discrete chassis with the first and second elastic belt laminates in a facing relationship from Figure 8 taken along line F-F.
Figure 9G is a view of two discrete absorbent articles advancing the machine direction MD from Figure 8 taken along line G-G.

### DETAILED DESCRIPTION OF THE INVENTION

The following term explanations may be useful in understanding the present disclosure:
"Absorbent article" is used herein to refer to consumer products whose primary function is to absorb and retain soils and wastes. "Diaper" is used herein to refer to an absorbent article generally worn by infants and incontinent persons about the lower torso. The term "disposable" is used herein to describe absorbent articles which generally are not intended to be laundered or otherwise restored or reused as an absorbent article (e.g., they are intended to be discarded after a single use and may also be configured to be recycled, composted or otherwise disposed of in an environmentally compatible manner).

An "elastic," "elastomer" or "elastomeric" refers to materials exhibiting elastic properties, which include any material that upon application of a force to its relaxed, initial length can stretch or elongate to an elongated length more than 10% greater than its initial length and will substantially recover back to about its initial length upon release of the applied force.

As used herein, the term "joined" encompasses configurations whereby an element is directly secured to another element by affixing the element directly to the other element, and configurations whereby an element is indirectly secured to another element by affixing the element to intermediate member(s) which in turn are affixed to the other element.

As used herein, the term "graphic" refers to printed areas of substrates. Graphics may include a color difference or transition of one or more colors and may define images or designs that are constituted by a figure (for example, a line(s)), a symbol or character), or the like. A graphic may include an aesthetic image or design that can provide certain benefit(s) when viewed. A graphic may be in the form of a photographic image. A graphic may also be in the form of a 1-dimensional (1-D) or 2-dimensional (2-D) bar code or a quick response (QR) bar code. A graphic design is determined by, for example, the color(s) used in the graphic (individual pure ink or spot colors as well as built process colors), the sizes of the entire graphic (or components of the graphic), the positions of the graphic (or components of the graphic), the movements of the graphic (or components of the graphic), the geometrical shapes of the graphic (or components of the graphics), the number of colors in the graphic, the variations of the color combinations in the graphic, the number of graphics printed, the disappearance of color(s) in the graphic, and the contents of text messages in the graphic.

It is to be appreciated that all graphics discussed herein may be in various different forms, shapes, and/or sizes than those depicted herein. It is also to be appreciated that the graphics described herein may be configured to be different graphics, standard graphics, custom graphics, and/or personalized graphics. "Different in terms of graphic design" means that graphics are intended to be different when viewed by users or consumers with normal attentions. Thus, two graphics having a graphic difference(s) which are unintentionally caused due to a problem(s) or an error(s) in a manufacture process, for example, are not different from each other in terms of graphic design. "Standard" or "standardized" refers to graphics, products, and/or articles that have the same aesthetic appearance without intending to be different from each other. The term "custom" or "customized" refers to graphics, products, and/or articles that are changed to suit a small demographic, region, purchaser, customer, or the like. Custom graphics may be selected from a set of graphics. For example, custom graphics may include animal depictions selected from groups of animals, such as farm animals, sea creatures, birds, and the like. In other examples, custom graphics may include nursery rhymes and the like. In one scenario, custom products or articles may be created by a purchaser of such products or articles wherein the purchaser selects graphics for the articles or products from a set of graphics offered by a manufacturer of such articles or products. Custom graphics may also include "personalized" graphics, which may be graphics created for a particular purchaser. For example, personalized graphics may include a person's name alone or in combination with a design.

"Longitudinal" means a direction running substantially perpendicular from a waist edge to a longitudinally opposing waist edge of an absorbent article when the article is in a flat out, uncontracted state, or from a waist edge to the bottom of the crotch, i.e. the fold line, in a bi-folded article. Directions within 45 degrees of the longitudinal direction are considered to be "longitudinal." "Lateral" refers to a direction running from a longitudinally extending side edge to a laterally opposing longitudinally extending side edge of an article and generally at a right angle to the longitudinal direction. Directions within 45 degrees of the lateral direction are considered to be "lateral."

The term "substrate" is used herein to describe a material which is primarily two-dimensional (i.e. in an XY plane) and whose thickness (in a Z direction) is relatively small (i.e. 1/10 or less) in comparison to its length (in an X direction) and width (in a Y direction). Non-limiting examples of substrates include a web, layer or layers or fibrous materials, nonwovens, films and foils such as polymeric films or metallic foils. These materials may be used alone or may comprise two or more layers laminated together. As such, a web is a substrate.

The term "nonwoven" refers herein to a material made from continuous (long) filaments (fibers) and/or discontinuous (short) filaments (fibers) by processes such as spunbonding, meltblowing, carding, and the like. Nonwovens do not have a woven or knitted filament pattern.

The term "machine direction" (MD) is used herein to refer to the direction of material flow through a process. In addition, relative placement and movement of material can be described as flowing in the machine direction through a process from upstream in the process to downstream in the process.

The term "cross direction" (CD) is used herein to refer to a direction that is generally perpendicular to the machine direction.

The term "pant" (also referred to as "training pant", "pre-closed diaper", "diaper pant", "pant diaper", and "pull-on diaper") refers herein to disposable absorbent articles having a continuous perimeter waist opening and continuous perimeter leg openings designed for infant or adult wearers. A pant can be configured with a continuous or closed waist opening and at least one continuous, closed, leg opening prior to the article being applied to the wearer. A pant can be preformed by various techniques including, but not limited to, joining together portions of the article using any refastenable and/or permanent closure member (e.g., seams, heat bonds, pressure welds, adhesives, cohesive bonds, mechanical fasteners, etc.). A pant can be preformed anywhere along the circumference of the article in the waist region (e.g., side fastened or seamed, front waist fastened or seamed, rear waist fastened or seamed).

The term "print density," which may also be referred to optical density, refers to the reflection density of printed matter, as measured with a spectrophotometer in accordance with the Method for Measuring Print Color and Print Density provided herein.

The present disclosure relates to absorbent articles and methods for assembling absorbent articles with components having printed graphics including zones of relatively high print densities and zones of relatively low print densities. More particularly, substrates and/or components to be incorporated into manufactured absorbent articles herein include graphics that may be positioned and/or printed in such a manner so as to functionally reduce noticeable visible results of imprecise and/or inconsistent manufacturing operations performed in areas where the graphics are located. For example, the substrates and/or components include graphics wherein the zones of relatively low print densities may be positioned in regions that are subject to bonding, cutting, and/or folding transformations during the assembly process. In addition, the zones of relatively high print densities may be positioned regions that may be more noticeable to consumers. For example, assembled diapers may include graphics with zones of relatively low print densities positioned along inner edges of front and/or back belts and/or leg openings, whereas the zones of relatively high print densities may be positioned closer to central portions of front and/or back waist regions. In some embodiments, assembled diapers may include components that are combined during manufacture, wherein each component includes graphics with zones of relatively low print densities positioned in areas where the components are combined. As such, the low print density zones may help reduce the noticeable results of imprecise placement of one printed component onto another printed component wherein the graphics on the separate components may otherwise appear disjointed and/or misaligned. Thus, the methods and apparatuses herein allow for the assemblage of substrates and/or components having graphics defining various designs and various colored areas printed thereon that extend over the entire area, or a relatively large area, of the assembled diapers that is visible when worn while maintaining desired aesthetic benefits on assembled diapers without sacrificing relatively high manufacturing speeds.

As previously mentioned, the processes and apparatuses discussed herein may be used in the manufacture of different types of absorbent articles. To help provide additional context to the subsequent discussion of the process embodiments, the following provides a general description of absorbent articles in the form of diaper pants that include belt substrates that may be assembled in accordance with the methods and apparatuses disclosed herein.

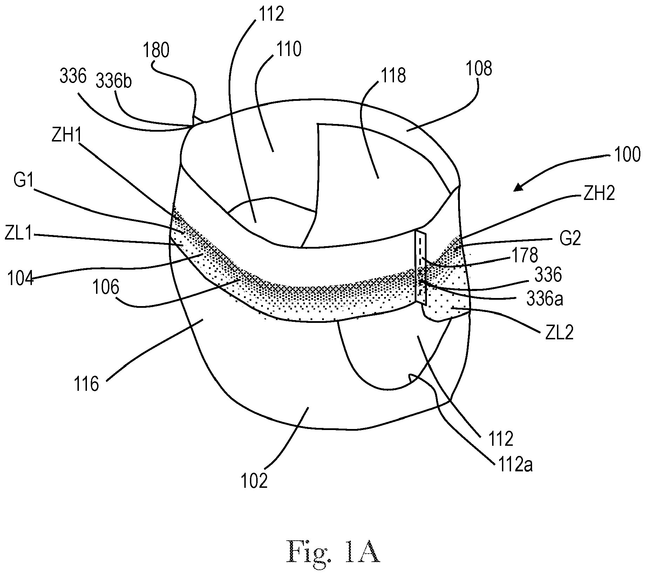

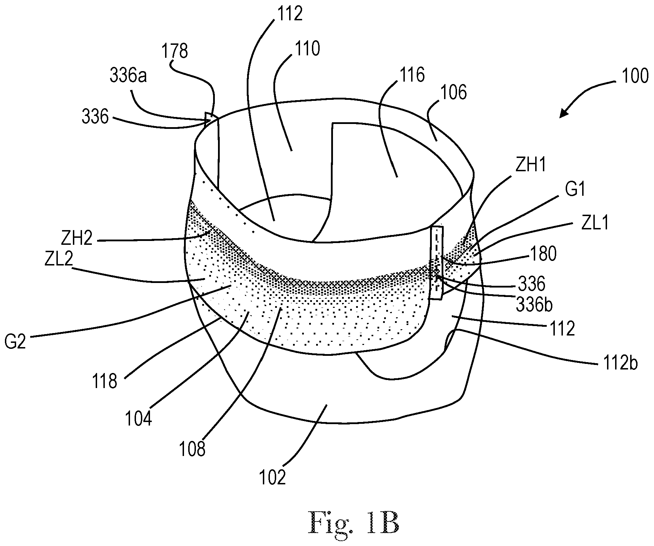

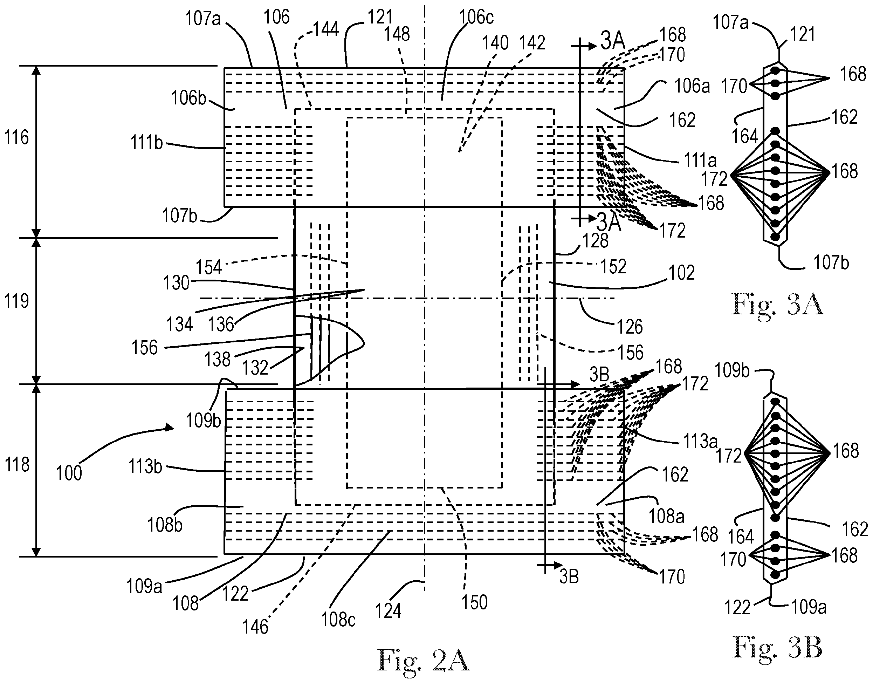

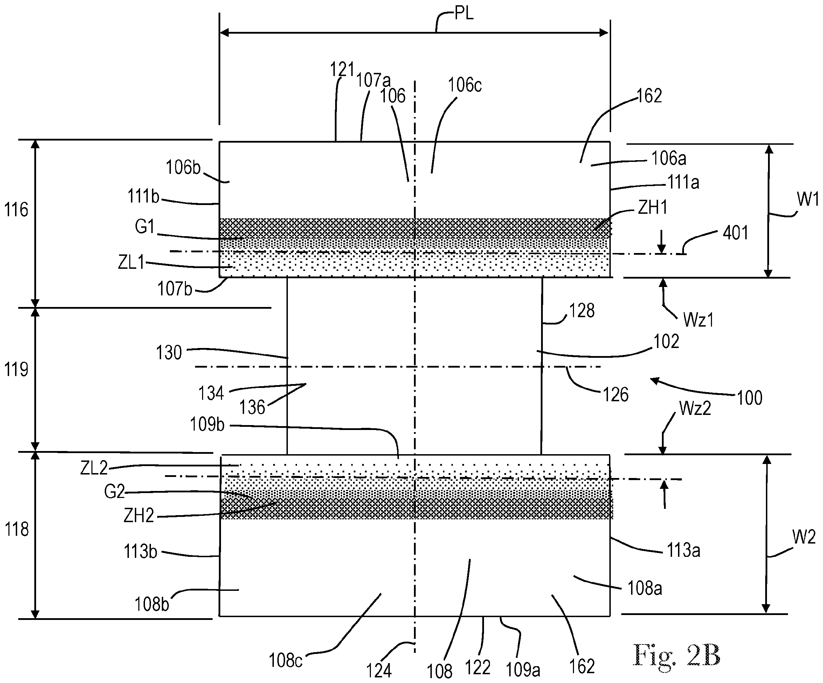

Figures 1A, 1B, 2A, and 2B show an example of a diaper pant 100 that may be assembled in accordance with the apparatuses and methods disclosed herein. In particular, Figures 1A and 1B show perspective views of a diaper pant 100 in a pre-fastened configuration, and Figures 2A and 2B show plan views of the diaper pant 100 with the portion of the diaper that faces away from a wearer oriented toward the viewer. The diaper pant 100 includes a chassis 102 and a ring-like elastic belt 104. As discussed below in more detail, a first elastic belt 106 and a second elastic belt 108 are bonded together to form the ring-like elastic belt 104.

With continued reference to Figures 2A and 2B, the diaper pant 100 and the chassis 102 each include a first waist region 116, a second waist region 118, and a crotch region 119 disposed intermediate the first and second waist regions. The first waist region 116 may be configured as a front waist region, and the second waist region 118 may be configured as back waist region. In some embodiments, the length of each of the front waist region, back waist region, and crotch region may be 1/3 of the length of the absorbent article 100. The diaper 100 may also include a laterally extending front waist edge 121 in the front waist region 116 and a longitudinally opposing and laterally extending back waist edge 122 in the back waist region 118. To provide a frame of reference for the present discussion, the diaper 100 and chassis 102 of Figures 2A and 2B are shown with a longitudinal axis 124 and a lateral axis 126. In some embodiments, the longitudinal axis 124 may extend through the front waist edge 121 and through the back waist edge 122. And the lateral axis 126 may extend through a first longitudinal or right side edge 128 and through a midpoint of a second longitudinal or left side edge 130 of the chassis 102.

As shown in Figures 1A, 1B, 2A, and 2B, the diaper pant 100 may include an inner, body facing surface 132, and an outer, garment facing surface 134. The chassis 102 may include a backsheet 136 and a topsheet 138. The chassis 102 may also include an absorbent assembly 140, including an absorbent core 142, disposed between a portion of the topsheet 138 and the backsheet 136. As discussed in more detail below, the diaper 100 may also include other features, such as leg elastics and/or leg cuffs to enhance the fit around the legs of the wearer.

As shown in Figures 2A and 2B, the periphery of the chassis 102 may be defined by the first longitudinal side edge 128, a second longitudinal side edge 130, a first laterally extending end edge 144 disposed in the first waist region 116, and a second laterally extending end edge 146 disposed in the second waist region 118. Both side edges 128 and 130 extend longitudinally between the first end edge 144 and the second end edge 146. As shown in Figure 2A, the laterally extending end edges 144 and 146 are located longitudinally inward from the laterally extending front waist edge 121 in the front waist region 116 and the laterally extending back waist edge 122 in the back waist region 118. When the diaper pant 100 is worn on the lower torso of a wearer, the front waist edge 121 and the back waist edge 122 may encircle a portion of the waist of the wearer. At the same time, the side edges 128 and 130 may encircle at least a portion of the legs of the wearer. And the crotch region 119 may be generally positioned between the legs of the wearer with the absorbent core 142 extending from the front waist region 116 through the crotch region 119 to the back waist region 118.

It is to also be appreciated that a portion or the whole of the diaper 100 may also be made laterally extensible. The additional extensibility may help allow the diaper 100 to conform to the body of a wearer during movement by the wearer. The additional extensibility may also help, for example, the user of the diaper 100, including a chassis 102 having a particular size before extension, to extend the front waist region 116, the back waist region 118, or both waist regions of the diaper 100 and/or chassis 102 to provide additional body coverage for wearers of differing size, i.e., to tailor the diaper to an individual wearer. Such extension of the waist region or regions may give the absorbent article a generally hourglass shape, so long as the crotch region is extended to a relatively lesser degree than the waist region or regions, and may impart a tailored appearance to the article when it is worn.

As previously mentioned, the diaper pant 100 may include a backsheet 136. The backsheet 136 may also define the outer surface 134 of the chassis 102. The backsheet 136 may be impervious to fluids (e.g., menses, urine, and/or runny feces) and may be manufactured in part from a thin plastic film, although other flexible liquid impervious materials may also be used. The backsheet 136 may prevent the exudates absorbed and contained in the absorbent core from wetting articles which contact the diaper 100, such as bedsheets, pajamas and undergarments. The backsheet 136 may also comprise a woven or nonwoven material, polymeric films such as thermoplastic films of polyethylene or polypropylene, and/or a multi-layer or composite materials comprising a film and a nonwoven material (e.g., having an inner film layer and an outer nonwoven layer). The backsheet may also comprise an elastomeric film. An example backsheet 136 may be a polyethylene film having a thickness of from about 0.012 mm (0.5 mils) to about 0.051 mm (2.0 mils). Exemplary polyethylene films are manufactured by Clopay Corporation of Cincinnati, Ohio, under the designation BR-120 and BR-121 and by Tredegar Film Products of Terre Haute, Ind., under the designation XP-39385. The backsheet 136 may also be embossed and/or matte-finished to provide a more clothlike appearance. Further, the backsheet 136 may permit vapors to escape from the absorbent core (i.e., the backsheet is breathable) while still preventing exudates from passing through the backsheet 136. The size of the backsheet 136 may be dictated by the size of the absorbent core 142 and/or particular configuration or size of the diaper 100.

Also described above, the diaper pant 100 may include a topsheet 138. The topsheet 138 may also define all or part of the inner surface 132 of the chassis 102. The topsheet 138 may be compliant, soft feeling, and non-irritating to the wearer's skin. It may be elastically stretchable in one or two directions. Further, the topsheet 138 may be liquid pervious, permitting liquids (e.g., menses, urine, and/or runny feces) to penetrate through its thickness. A topsheet 138 may be manufactured from a wide range of materials such as woven and nonwoven materials; apertured or hydroformed thermoplastic films; apertured nonwovens, porous foams; reticulated foams; reticulated thermoplastic films; and thermoplastic scrims. Woven and nonwoven materials may comprise natural fibers such as wood or cotton fibers; synthetic fibers such as polyester, polypropylene, or polyethylene fibers; or combinations thereof. If the topsheet 138 includes fibers, the fibers may be spunbond, carded, wet-laid, meltblown, hydroentangled, or otherwise processed as is known in the art.

Topsheets 138 may be selected from high loft nonwoven topsheets, apertured film topsheets and apertured nonwoven topsheets. Apertured film topsheets may be pervious to bodily exudates, yet substantially non-absorbent, and have a reduced tendency to allow fluids to pass back through and rewet the wearer's skin. Exemplary apertured films may include those described in U.S. Patent Nos. 5,628,097; 5,916,661; 6,545,197; and 6,107,539.

As mentioned above, the diaper pant 100 may also include an absorbent assembly 140 that is joined to the chassis 102. As shown in Figure 2A, the absorbent assembly 140 may have a laterally extending front edge 148 in the front waist region 116 and may have a longitudinally opposing and laterally extending back edge 150 in the back waist region 118. The absorbent assembly may have a longitudinally extending right side edge 152 and may have a laterally opposing and longitudinally extending left side edge 154, both absorbent assembly side edges 152 and 154 may extend longitudinally between the front edge 148 and the back edge 150. The absorbent assembly 140 may additionally include one or more absorbent cores 142 or absorbent core layers. The absorbent core 142 may be at least partially disposed between the topsheet 138 and the backsheet 136 and may be formed in various sizes and shapes that are compatible with the diaper. Exemplary absorbent structures for use as the absorbent core of the present disclosure are described in U.S. Patent Nos. 4,610,678; 4,673,402; 4,888,231; and 4,834,735.

Some absorbent core embodiments may comprise fluid storage cores that contain reduced amounts of cellulosic airfelt material. For instance, such cores may comprise less than about 40%, 30%, 20%, 10%, 5%, or even 1% of cellulosic airfelt material. Such a core may comprises primarily absorbent gelling material in amounts of at least about 60%, 70%, 80%, 85%, 90%, 95%, or even about 100%, where the remainder of the core comprises a microfiber glue (if applicable). Such cores, microfiber glues, and absorbent gelling materials are described in U.S. Patent Nos. 5,599,335; 5,562,646; 5,669,894; and 6,790,798 as well as U.S. Patent Publication Nos. 2004/0158212 and 2004/0097895.

As previously mentioned, the diaper 100 may also include elasticized leg cuffs 156. It is to be appreciated that the leg cuffs 156 can be and are sometimes also referred to as leg bands, side flaps, barrier cuffs, elastic cuffs or gasketing cuffs. The elasticized leg cuffs 156 may be configured in various ways to help reduce the leakage of body exudates in the leg regions. Example leg cuffs 156 may include those described in U.S. Patent Nos. 3,860,003; 4,909,803; 4,695,278; 4,795,454; 4,704,115; 4,909,803; and U.S. Patent Publication No. 2009/0312730 A1.

As mentioned above, diaper pants may be manufactured with a ring-like elastic belt 104 and provided to consumers in a configuration wherein the front waist region 116 and the back waist region 118 are connected to each other as packaged, prior to being applied to the wearer. As such, diaper pants may have a continuous perimeter waist opening 110 and continuous perimeter leg openings 112 such as shown in Figures 1A and 1B. The ring-like elastic belt may be formed by joining a first elastic belt to a second elastic belt with a permanent side seam or with an openable and reclosable fastening system disposed at or adjacent the laterally opposing sides of the belts.

As previously mentioned, the ring-like elastic belt 104 may be defined by a first elastic belt 106 connected with a second elastic belt 108. As shown in Figures 2A and 2B, the first elastic belt 106 extends between a first longitudinal side edge 111a and a second longitudinal side edge 111b and defines first and second opposing end regions 106a, 106b and a central region 106c. And the second elastic 108 belt extends between a first longitudinal side edge 113a and a second longitudinal side edge 113b and defines first and second opposing end regions 108a, 108b and a central region 108c. The distance between the first longitudinal side edge 111a and the second longitudinal side edge 111b defines the pitch length, PL, of the first elastic belt 106, and the distance between the first longitudinal side edge 113a and the second longitudinal side edge 113b defines the pitch length, PL, of the second elastic belt 108. The central region 106c of the first elastic belt is connected with the first waist region 116 of the chassis 102, and the central region 108c of the second elastic belt 108 is connected with the second waist region 116 of the chassis 102. As shown in Figures 1A and 1B, the first end region 106a of the first elastic belt 106 is connected with the first end region 108a of the second elastic belt 108 at first side seam 178, and the second end region 106b of the first elastic belt 106 is connected with the second end region 108b of the second elastic belt 108 at second side seam 180 to define the ring-like elastic belt 104 as well as the waist opening 110 and leg openings 112.

It is to be appreciated that the first and second elastic belts may define various pitch lengths PL. For example, in some embodiments, the pitch lengths PL of the first and/or second elastic belts may be about 300 mm to about 1100 mm.

As shown in Figures 2A, 3A, and 3B, the first elastic belt 106 also defines an outer laterally extending edge 107a and an inner laterally extending edge 107b, and the second elastic belt 108 defines an outer laterally extending edge 109a and an inner laterally extending edge 109b. As such, a perimeter edge 112a of one leg opening may be defined by portions of the inner laterally extending edge 107b of the first elastic belt 106, the inner laterally extending edge 109b of the second elastic belt 108, and the first longitudinal or right side edge 128 of the chassis 102. And a perimeter edge 112b of the other leg opening may be defined by portions of the inner laterally extending edge 107b, the inner laterally extending edge 109b, and the second longitudinal or left side edge 130 of the chassis 102. The outer laterally extending edges 107a, 109a may also define the front waist edge 121 and the laterally extending back waist edge 122 of the diaper pant 100. The first elastic belt and the second elastic belt may also each include an outer, garment facing layer 162 and an inner, wearer facing layer 164. It is to be appreciated that the first elastic belt 106 and the second elastic belt 108 may comprise the same materials and/or may have the same structure. In some embodiments, the first elastic belt 106 and the second elastic belt may comprise different materials and/or may have different structures. It should also be appreciated that the first elastic belt 106 and the second elastic belt 108 may be constructed from various materials. For example, the first and second belts may be manufactured from materials such as plastic films; apertured plastic films; woven or nonwoven webs of natural materials (e.g., wood or cotton fibers), synthetic fibers (e.g., polyolefins, polyamides, polyester, polyethylene, or polypropylene fibers) or a combination of natural and/or synthetic fibers; or coated woven or nonwoven webs. In some embodiments, the first and second elastic belts include a nonwoven web of synthetic fibers, and may include a stretchable nonwoven. In other embodiments, the first and second elastic belts include an inner hydrophobic, non-stretchable nonwoven material and an outer hydrophobic, non-stretchable nonwoven material.

The first and second elastic belts 106, 108 may also each include belt elastic material interposed between the outer substrate layer 162 and the inner substrate layer 164. The belt elastic material may include one or more elastic elements such as strands, ribbons, films, or panels extending along the lengths of the elastic belts. As shown in Figures 2A, 3A, and 3B, the belt elastic material may include a plurality of elastic strands 168 which may be referred to herein as outer, waist elastics 170 and inner, waist elastics 172. Elastic strands 168, such as the outer waist elastics 170, may continuously extend laterally between the first and second opposing end regions 106a, 106b of the first elastic belt 106 and between the first and second opposing end regions 108a, 108b of the second elastic belt 108. In some embodiments, some elastic strands 168, such as the inner waist elastics 172, may be configured with discontinuities in areas, such as for example, where the first and second elastic belts 106, 108 overlap the absorbent assembly 140. In some embodiments, the elastic strands 168 may be disposed at a constant interval in the longitudinal direction. In other embodiments, the elastic strands 168 may be disposed at different intervals in the longitudinal direction. The belt elastic material in a stretched condition may be interposed and joined between the uncontracted outer layer and the uncontracted inner layer. When the belt elastic material is relaxed, the belt elastic material returns to an unstretched condition and contracts the outer layer and the inner layer. The belt elastic material may provide a desired variation of contraction force in the area of the ring-like elastic belt. It is to be appreciated that the chassis 102 and elastic belts 106, 108 may be configured in different ways other than as depicted in Figure 2A. The belt elastic material may be joined to the outer and/or inner layers continuously or intermittently along the interface between the belt elastic material and the inner and/or outer belt layers.

In some configurations, the first elastic belt 106 and/or second elastic belt 108 may define curved contours. For example, the inner lateral edges 107b, 109b of the first and/or second elastic belts 106, 108 may include non-linear or curved portions in the first and second opposing end regions. Such curved contours may help define desired shapes to leg opening 112, such as for example, relatively rounded leg openings. In addition to having curved contours, the elastic belts 106, 108 may include elastic strands 168, 172 that extend along non-linear or curved paths that may correspond with the curved contours of the inner lateral edges 107b, 109b.

As previously mentioned, the diaper pant 100 may include one or more graphics. And such graphics may include zones of relatively high print densities, referred to herein as "high intensity zones," and zones of relatively low print densities, referred to herein as "low intensity zones." As discussed above, the diaper components may include graphics positioned and/or printed in such a manner so as to reduce noticeable visible results of imprecise and/or inconsistent manufacturing operations performed in areas where the printing is located. Thus, the high intensity zones may be positioned in regions of the diaper that may be more noticeable to consumers. And the low intensity zones may be positioned in regions that are subject to combining, cutting, and/or folding transformations during the assembly process, such as inner belt edge and/or leg opening regions. With respect to the graphics discussed herein, each zone comprises a maximum print density, and the maximum print density of the low intensity zone is greater than zero and less than the maximum print density of the high intensity zone. For example, in some embodiments, the maximum print density of the high intensity zone may be at least about 0.3; 0.4; 0.5; 0.8; 1.0; or 1.2. And in some embodiments, the maximum print density of the low intensity zone may be greater than zero and less than or equal to about 0.3, 0.2, 0.15; or 0.1. In some embodiments, the maximum print density of the low intensity zone may be less than or equal to about 30% of the maximum print density of the high intensity zone. In some embodiments, the maximum print density of the low intensity zone may be less than or equal to about 25% of the maximum print density of the high intensity zone. In some embodiments, the maximum print density of the low intensity zone may be less than or equal to about 10% of the maximum print density of the high intensity zone. In addition, the graphics may be printed so as to fade from the high intensity zone to the low intensity zone. As used herein, the term "fade" means a visible gradual change in color hue, brightness, lightness, chroma, and/or saturation, for example, when a graphic fades from an area having a relatively high print density to an area having a relatively low print density.

It is to be appreciated that the graphics described herein may be printed in various ways and may be printed by various types of printing accessories, such as ink jet, flexography, and/or gravure printing processes. Ink-jet printing is a non-impact dot-matrix printing technology in which droplets of ink are jetted from a small aperture directly to a specified position on a media to create a graphic. Two examples of inkjet technologies include thermal bubble or bubble jet and piezoelectric. Thermal bubble uses heat to apply to the ink, while piezoelectric uses a crystal and an electric charge to apply the ink. In some configurations, the printing stations may include a corona treater, which may be positioned upstream of the printer. The corona treater may be configured to increase the surface energy of the surface of the substrate to be printed. In some configurations, the printing stations may also include an ink curing apparatus. In some configurations, the ink curing apparatus may be in the form of an ultraviolet (UV) light source that may include one or more ultraviolet (UV) lamps, which may be positioned downstream of the printer to help cure inks deposited onto the substrate from the printer to form the graphics. In some configurations, the ink curing apparatus may also include an infrared (IR) dryer light source that may include one or more infrared (IR) lamps, which may be positioned downstream of the printer to help dry water-based or solvent-based inks deposited onto the substrate to form the graphics. In some configurations, the ink curing apparatus may include an electron beam (EB or e-beam) generator that may include one or more e-beam electrodes, which may be positioned downstream of the printer to help cure inks deposited onto the substrate from the printer to form the graphics.

Figures 1A, 1B, and 2B show an example diaper pant 100 with printed graphics G1, G2 on the first elastic belt 106 and the second elastic belt 108, wherein the first graphic G1 includes a high intensity zone ZH1 and a low intensity zone ZL1. And the second graphic G2 includes a high intensity zone ZH2 and a low intensity zone Zl2. As shown in Figure 2B, the low intensity zone ZL1 is positioned along the laterally extending inner edge 107b of the first belt 106, and the low intensity zone ZL2 is positioned along the laterally extending inner edge 109b of the second belt 108. In addition, the high intensity zones ZH1, ZH2 are positioned away from the inner edges 107b, 109b of the first and second belts 106, 108.

With continued reference to Figures 1A, 1B, and 2B, the low intensity zone ZL1 of the graphic G1 on the front belt 106 is positioned between the high intensity zone ZH1 and the inner edge 107b of the first belt 106. And the low intensity zone ZL2 of the graphic G2 on the second belt 108 is positioned between the high intensity zone ZH2 and the inner edge 109b of the second belt 108. For the purposes of clarity, dashed lines 401 are shown in Figure 2B to represent example boundaries between the high intensity zones ZH1, ZH2 and the low intensity zones ZL1, ZH2, respectively. It is to be appreciated that such boundaries between the high intensity zones and the low intensity zones can also be curved, angled, and/or straight. As shown in Figure 2B, the low intensity zone ZL1 of the graphic G1 on the front belt 106 may extend from the high intensity zone ZH1 entirely to the inner edge 107b, and the low intensity zone ZL2 of the graphic G2 on the back belt 108 may extend from the high intensity zone ZH2 entirely to the inner edge 109b. It is to be appreciated that in some embodiments, one or all of the low intensity zones ZL1, ZL2 may not extend all the way to the outer edges 107a, 109a. As also shown in Figure 2B, the low intensity zone ZL1 of the graphic G1 on the front belt 106 may extend contiguously from the first longitudinal side edge 111a to the second longitudinal side edge 111b, and the low intensity zone ZL of the graphic G2 on the back belt 108 may extend contiguously from the first longitudinal side edge 113a to the second longitudinal side edge 113b. It is to be appreciated that in some embodiments, one or all the low intensity zones ZL1, ZL2 may not extend all the way to one of or both of the longitudinal side edges 111a, 111b on the front belt 106 and/or all the way to one of or both of the longitudinal side edges 113a, 113b on the back belt 108. In some embodiments, either or both the high intensity zones ZH1, ZH2 and/or either or both the low intensity zones ZL1, ZL2 may extend continuously for less than or equal to about 40% of the pitch length PL of the diaper pant 100.

As previously discussed, the low intensity zones ZL1, ZL2 are positioned in regions of the diapers 100 that may be subject to various cutting and/or folding transformations during the assembly process so as to reduce noticeable visible results of imprecisions and/or inconsistencies of such transformations. Thus, it is also to be appreciated that the low intensity zones ZL1, ZL2 discussed herein may be devoid of additional graphics. As such, it may be desirable in some embodiments to manufacture absorbent articles with graphics having a high intensity zone and a low intensity zone wherein the low intensity zone is devoid of any other printed graphics or the like.

As shown in Figure 2B, the distance between the outer laterally extending edge 107a and the inner laterally extending edge 107b may define a width, W1, of the first belt 106. And the distance between the outer laterally extending edge 109a and the inner laterally extending edge 109b may define a width, W2, of the second belt 108. As shown in Figure 2B, the low intensity zone ZL1 of the first graphic G1 may also define a width Wz1 along the first belt 106, and the low intensity zone ZL of the second graphic G2 may define a width Wz2 along the second belt 108. It is to be appreciated that widths Wz1, Wz2 of the low intensity zones ZL1, ZL2 may vary. In some embodiments, the widths Wz1, Wz2 of the low intensity zones ZL1, ZL2 may be from about 4 mm to about 15 mm. In some embodiments, the widths W1 and/or W2 of the first and second belts 106, 108 may be from about 120 mm to about 300 mm. In some embodiments, the widths Wz1, Wz2 may be expressed in terms relative to the widths W1, W2 of the first and second belts 106, 108. For example, in some embodiments, the widths W1, W2 of the first and/or second belts 106, 108 may be about 8 to about 75 times the widths Wz1, Wz2 of the low intensity zones ZL1, ZL2 of graphics G1 and/or G2, respectively. In some embodiments, the width Wz1 may be less than or equal to about 10% of width W1, and/or the width Wz2 may be less than or equal to about 10% of width W2.

As previously mentioned, substrates and/or components that may be incorporated into manufactured absorbent articles, such as shown in Figure 2B, include graphics that may be positioned and/or printed in such a manner so as to reduce noticeable visible results of imprecise and/or inconsistent manufacturing operations performed in areas where the printing is located. It is to be appreciated that various apparatuses and methods according to the present disclosure may be utilized to assemble various components of pre-fastened pant diapers 100 described herein. For example, Figure 4 shows a schematic view of a converting apparatus 300 adapted to manufacture pant diapers 100. The method of operation of the converting apparatus 300 may be described with reference to the various components of pant diapers 100 described above and shown in Figures 1A, 1B, 2A, and 2B. Although the following methods are provided in the context of the diaper 100 shown in Figures 1A, 1B, 2A, and 2B, it is to be appreciated that various embodiments of diaper pants can be manufactured according to the methods disclosed herein, such as for example, the absorbent articles disclosed in U.S. Patent No. 7,569,039; U.S. Patent Publication Nos. 2005/0107764 A1, 2012/0061016 A1, and 2012/0061015 A1, which are all hereby incorporated by reference herein.

As described in more detail below, the converting apparatus 300 shown in Figure 4 operates to advance first and second elastic belt laminates 406, 408 along a machine direction MD. In addition, a continuous length of chassis assemblies 302 are advanced in a machine direction MD and cut into discrete chassis 102 such that the longitudinal axis 124 of each chassis 102 is parallel with the machine direction MD. The discrete chassis 102 are then turned to advance the discrete chassis 102 along the machine direction MD such that the lateral axis 126 of each chassis 102 is parallel with the machine direction MD. The discrete chassis 102 are also spaced apart from each other along the machine direction MD. Opposing waist regions 116, 118 of the spaced apart chassis 102 are then connected with continuous lengths of advancing first and second elastic belt laminates 406, 408. The chassis 102 may then be folded along the lateral axis, or parallel to the lateral axis, to bring the first and second elastic belt laminates 406, 408 into a facing relationship, and the first and second elastic belt laminates are bonded together with laterally opposing bonds 336. As discussed in more detail below, the first and second elastic belt laminates may be bonded together with adjacent bonds 336a, 336b intermittently spaced along the machine direction MD. It is to be appreciated that the bonds 336a, 336b may be configured as permanent and/or refastenable bonds. And each bond 336a, 336b may be a discrete bond site extending contiguously in a cross direction CD across a width of the first and second elastic belt laminates and/or may include a plurality of relatively small, discrete bond sites arranged in the cross direction. The first and second continuous elastic laminates 406, 408 are then cut in the cross direction CD between adjacent bonds 336a, 336b to create discrete pant diapers 100, such as shown in Figures 1A and 1B.

As shown in Figure 4, a first continuous substrate layer in the form of a continuous length of outer layer belt substrate 162; a second continuous substrate layer in the form of a continuous length of inner layer belt substrate 164; and elastics 168 are combined to form a continuous elastic laminate 402 in the form of a belt material. More particularly, continuous lengths of outer layer belt substrate 162, inner layer belt substrate 164, outer elastic strands 170 and inner elastic strands 172 are advanced in a machine direction MD and combined at nip rolls 502 to form the continuous elastic laminate 402. Before entering the nip rolls 502, the outer layer belt substrate 162 and/or the inner layer belt substrate 164 may be printed with graphics having high intensity zones and low intensity zones. It is to be appreciated that the graphic printing may be done during the assembly process and/or may done separate to the assembly process, such as for example, printing the substrates off line wherein the printed substrates may be stored until needed for production.

As shown in Figures 4, 5A1, and 5A2, the outer belt substrate 162 includes first surface 162a and an opposing second surface 162b, and defines a width W in the cross direction CD between opposing first and second longitudinal edges 163a, 163b. And the inner belt substrate 164 includes first surface 164a and an opposing second surface 164b, and defines a width in the cross direction CD between opposing first and second longitudinal edges 165a, 165b. As shown in Figure 5A2, the width W of the outer belt substrate 162 may be greater than the width of the inner belt substrate 164. And the width W of the outer belt substrate 162 may also define the width W of the elastic laminate 402. It is to be appreciated that in some embodiments, the width of the inner belt substrate 164 may be the same as or greater than the width of the outer belt substrate 162.

With continued reference to Figure 4, before entering the nip rolls 502, the outer elastic strands 170 and inner elastic strands 172 are stretched in the machine direction MD. In addition, adhesive 504 may be applied to the elastic strands 170, 172 as well as either or both of the continuous lengths of outer layer belt substrate 162 and inner layer belt substrate 164 before entering nip rolls 502. As such, the elastic strands 168 are bonded between the first surface 162a of the outer layer belt substrate 162 and the first surface 164a of inner layer belt substrate 164 at the nip rolls 502. Further, adhesive 504 may be applied intermittently along the lengths of the inner elastic strands 172 and/or intermittently along the length of either or both of the continuous lengths of outer layer belt substrate 162 and inner layer belt substrate 164 before entering nip rolls 502. As such, the inner elastic strands 172 may be intermittently bonded to either or both of the continuous lengths of outer layer belt substrate 162 and inner layer belt substrate 164 along the machine direction MD. Thus, the continuous elastic laminate 402 may include non-bonded regions intermittently spaced between bonded regions along the machine direction MD, wherein the inner elastic strands 172 are not bonded to either the outer layer belt substrate 162 or inner layer belt substrate 164 in the non-bonded regions. And the inner elastic strands 172 are bonded to the outer layer belt substrate 162 and/or inner layer belt substrate 164 in the bonded regions. As such, the elastic strands 172 may be severed in the non-bonded regions in a subsequent process step. Although Figure 4 shows an embodiment wherein the continuous elastic laminate 402 is formed by combining continuous lengths of outer layer belt substrate 162 and inner layer belt substrate 164 with elastic strands 168, it is to be appreciated the continuous elastic laminate 402 can be formed in various ways, such as disclosed in U.S. Patent No. 8,440,043 and U.S. Patent Publication Nos. 2013/0255861 A1; 2013/0255862 A1; 2013/0255863 A1; 2013/0255864 A1; and 2013/0255865 A1.

As shown in Figures 4 and 5A1, the outer belt substrate 162 advances in the machine direction and may include graphics G printed on the first surface 162a of the outer layer belt substrate 162. As shown in Figure 5A1, although the graphics G are printed on the first surface 162a of the outer layer belt substrate 162, the graphics G may be visible through the second surface 162b. It is also to be appreciated that the graphics G may be printed on either or both the first and second surfaces 162a, 162b of the outer belt substrate 162. It is also to be appreciated that graphics may be printed on either or both the first and second surfaces 164a, 164b of the inner belt substrate 164.

As shown in Figure 5A1, the graphics G extend in the machine direction MD and includes a low intensity zone ZL, a first high intensity zone ZH1, and a second high intensity zone ZH2. The low intensity zone ZL is a central zone positioned between the laterally opposing first high intensity zone ZH1 and the second high intensity zone ZH2. For the purposes of clarity, dashed lines 401 are shown in Figure 5A1 to represent example boundaries between the high intensity zones ZH1, ZH2 and the low intensity zone ZL. It is to be appreciated that such boundaries between the high intensity zones ZH1, ZH2 and the low intensity zone ZL can also be curved, angled, and/or straight. As shown in Figure 5A1, the low intensity zone ZL of the graphics G defines a width, Wz, in the cross direction CD. It is to be appreciated that width Wz of the low intensity zone ZL may vary. In some embodiments, the width Wz may be from about 8 mm to about 30 mm. In some embodiments, the width W of the belt substrate 162 and/or elastic laminate 402 may be from about 240 mm to about 600 mm. In some embodiments, the width Wz may also be expressed in terms relative to the width W of the belt substrate 162 and/or the elastic laminate 402. For example, in some embodiments, the width W of the outer belt substrate 162 and/or the elastic laminate 402 may be about 8 to about 75 times the width Wz of the low intensity zone ZL. In some embodiments, the width Wz may be less than or equal to about 10% of the width W. Although the low intensity zone ZL of the graphics G is depicted as extending contiguously in the machine direction MD, it is to be appreciated that the low intensity zone ZL of the graphics G may be defined by discrete lengths extending in the machine direction MD. It is to also to be appreciated that the graphics G may be printed to have differing designs from each other along the machine direction MD and/or cross direction CD. Also shown in Figure 5A1, the outer belt substrate 162, and thus the elastic laminate 402, may include first and second outer longitudinal regions 166a, 166b separated in the cross direction CD by a central region 166c. And the central zone ZL of the graphic G may be positioned entirely within the central region 166c of the elastic laminate 402. It is to be appreciated the widths of the regions 166a, 166b, 166c may vary. For example, in some embodiments, the central region 166c may be about 33% of the width W of the elastic laminate 402. In some embodiments, the first and second outer longitudinal regions 166a, 166b and/or the central region may each be about 1/3 of the width W of the elastic laminate 402.

With continued reference to Figures 4, 5A2, and 5B, from the nip rolls 502 the continuous elastic laminate 402 advances in the machine direction MD to a cutter 506 that cuts the continuous elastic laminate 402 into two continuous elastic belt laminates, referred to as a first elastic belt laminate 406 and a second elastic belt laminate 408. In particular, the cutter 506 operates to cut the elastic laminate 402 along the machine direction and through the central zone ZL to form the first continuous elastic laminate 406 and the second continuous elastic laminate 408. As such, the cutter 506 also operates to divide the graphic G into a first graphic G1 and a second graphic G2, wherein the first graphic is positioned on the first elastic laminate 406 and the second graphic G2 is positioned on the second elastic laminate 408. As shown in Figure 5B, the first elastic laminate 406 includes an inner longitudinal edge 107b and an outer longitudinal edge 107a, and the second elastic laminate 406 includes an inner longitudinal edge 109b and an outer longitudinal edge 109a. A first portion ZL1 of the central zone ZL defines the low intensity zone of the first graphic G1 and extends along inner longitudinal edge 107b of the first continuous elastic laminate 406. And a second portion ZL2 of the central zone ZL defines the low intensity zone of the second graphic G2 and extends along inner longitudinal edge 109b of the second continuous elastic laminate 408. As previously mentioned, the cutter 506 cuts through the central zone ZL of the graphic G, which is a low intensity zone. Thus, cutting the elastic laminate 402 in the machine direction through the low intensity zone ZL may help reduce noticeable visible results of an imprecise and/or crooked cut line that defines the inner longitudinal edges 107b, 109b of the first and second elastic laminates 406, 408.

As shown in Figure 5B, the first belt laminate 406 extends between the outer longitudinal edge 107a and the inner longitudinal edge 107b to define a width W1 in the cross direction CD. And the second belt laminate 408 extends between the outer longitudinal edge 109a and the inner longitudinal edge 109b to define a width W2 in the cross direction CD. In addition, the low intensity zone ZL1 of the first graphic G1 may define a width Wz1 in the cross direction CD, and the low intensity zone ZL2 of the second graphic G2 may define a width Wz2 in the cross direction CD. It is to be appreciated that W2 may be greater than W1. It is also to be appreciated that in some configurations, W1 may be equal to or greater than W2. In some embodiments, the widths W1 and/or W2 may be from about 120 mm to about 300 mm. In addition, the widths Wz1, Wz2 of the low intensity zone ZL1, ZL2 may be expressed in terms relative to the width W1, W2 of the first and second belt laminates 406, 408, respectively. For example, in some embodiments, the widths W1, W2 of the first and/or second belt laminates 406, 408 may be about 8 to about 75 times the widths Wz1, Wz2 of the low intensity zones ZL1, ZL2 of graphics G1 and/or G2 as viewed from same side of the first and/or second belt laminates 406, 408. In some embodiments, the width Wz1 may be less than or equal to about 10% of width W1, and/or the width Wz2 may be less than or equal to about 10% of width W2.

As shown in Figure 5B, the high intensity zone ZH1 of the first graphics G1 may not extend entirely in the cross direction CD from the low intensity zone ZL1 to the outer longitudinal edge 107a. And the high intensity zone ZH2 of the second graphics G2 may not extend entirely in the cross direction CD from the low intensity zone ZL2 to the outer longitudinal edge 109a. It is to be appreciated that in some embodiments, the high intensity zone ZH1 of the first graphics G1 may extend entirely in the cross direction CD from the low intensity zone ZL1 to the outer longitudinal edge 107a, and the high intensity zone ZH2 of the second graphics G2 may extend entirely in the cross direction CD from the low intensity zone ZL2 to the outer longitudinal edge 109a.

It is also to be appreciated that the cutter 506 may be configured in various ways. For example, in some embodiments the cutter 506 may be a slitter or a die cutter that separates the belt material into two continuous belt substrates with either a straight line cut and/or a curved line cut. The cutter 506 may also be configured as a perforator that perforates the belt material with a line of weakness and wherein the belt material is separated along the line of weakness in a later step. From the cutter 506, the first and second belt laminates 406, 408 advance through a diverter 508 that separates the first and second belt substrates from each other in the cross direction CD, such as shown in Figure 5B. The elastic strands 170, 172, and thus, the continuous length of first and second belt laminates 406, 408 are maintained in a stretched condition while advancing along the machine direction MD.

In some embodiments, the cut line through the elastic laminate 402 created by the cutter 506 may define the inner edge 107b of the first belt laminate 406 and/or the inner edge 109b of the second belt laminate 408. In some embodiments, the first belt laminate 406 and/or the second belt laminate 408 may advance from the cutter 506 to a folding apparatus adapted that folds the cut edges of the first and/or second belt laminates created by the cutter 506. As such, the inner edge 107b of the first belt laminate 406 and/or the inner edge 109b of the second belt laminate 408 may be defined by a fold line extending along the machine direction MD.

It is to be appreciated that the diverter 508 may be configured in various ways. For example, in some embodiments, the diverter 508 may include turn bars angled at 45 degrees or some other angle with respect to the machine direction. In some embodiments, the diverter may include cambered rollers. It is to be appreciated that the front and back belts may be formed by separate continuous lengths of belt material similar to the description above and as such would not required the slitting step or the diverting step. And in some embodiments, the front and back belts may be formed by slitting the outer belt substrate 162 and the inner belt substrate 164 along the machine direction MD before being combined with the elastic material 168.

In some embodiments, the diverter 508 may include a pivot or tracking table, such as for example, the FIFE-500 Web Guiding System, by Maxcess-FIFE Corporation, which can adjust the positions of the continuous length of first and second belt laminates 406, 408 in the cross direction CD. Other suitable pivot or tracking tables are available from Erhardt & Leimer, Inc. The diverter may also include instrumentation and web edge control features that allow for precise active control of the substrate positions.

As previously mentioned, the first belt laminate 406 is separated in the cross direction CD from the second belt laminate 408 to define a gap between the inner longitudinal edge 107b of the first belt laminate 406 and the inner longitudinal edge 109b of the second belt laminate 408. As discussed in more detail below, the first and second belt laminate 406, 408 advance from the diverter 508 to a nip 316 between the carrier apparatus 308 and a roll 318 to be combined with discrete chassis 102.

Referring now to Figures 4 and 5C, a continuous length of chassis assemblies 302 are advanced in a machine direction MD and define a width in a cross direction CD. The continuous length of chassis assemblies 302 may include absorbent assemblies 140 sandwiched between topsheet material 138 and backsheet material 136, leg elastics, barrier leg cuffs and the like. As shown in Figure 5C, portion of the chassis assembly is cut-away to show a portion of the topsheet material 138 and an absorbent assembly 140. The continuous length of chassis assemblies 302 advance to a carrier apparatus 308 and are cut into discrete chassis 102 with knife roll 306, while advancing in the orientation shown in Figure 5D1, wherein the longitudinal axis 124 of each chassis 102 is generally parallel with the machine direction MD.

After the discrete absorbent chassis 102 are cut by the knife roll 306, the carrier apparatus 308 rotates and advances the discrete chassis 102 in the machine direction MD in the orientation shown in Figure 5D1. While the chassis 102 shown in Figure 5D1 is shown with the second laterally extending end edge 146 as a leading edge and the first laterally extending end edge 144 as the trailing edge, it is to be appreciated that in other embodiments, the chassis 102 may be advanced in other orientations. For example, the chassis may be oriented such that the second laterally extending end edge 146 is a trailing edge and the first laterally extending end edge 144 is a leading edge. The carrier apparatus 308 also rotates while at the same time changing the orientation of the advancing chassis 102. In changing the chassis orientation, the carrier apparatus 308 may turn each chassis 102 such that the lateral axis 126 of the chassis 102 is parallel or generally parallel with the machine direction MD, such as shown in Figure 5D2. The carrier apparatus 308 may also change the speed at which the chassis 102 advances in the machine direction MD to a different speed. Figure 5D2 shows the orientation of the chassis 102 on the carrier apparatus 308 while advancing in the machine direction MD. More particularly, Figure 5D2 shows the chassis 102 with the lateral axis 126 of the chassis 102 generally parallel with the machine direction MD, and wherein the second longitudinal side edge 130 is the leading edge and the first longitudinal side edge 128 is the trailing edge. It is to be appreciated that various forms of carrier apparatuses may be used with the methods herein, such as for example, the carrier apparatuses disclosed in U.S. Patent No. 7,587,966 and U.S. Patent Publication Nos. 2013/0270065 A1; 2013/0270069 A1; 2013/0270066 A1; and 2013/0270067 A1. In some embodiments, the carrier apparatus 308 may rotate at a variable angular velocity that may be changed or adjusted by a controller in order to change the relative placement of the chassis 102 and the advancing belt laminates 406, 408.

As discussed below with reference to Figures 4, 5E1, 5E2, 5F, and 5G, the chassis 102 are transferred from the carrier apparatus 308 and combined with advancing, continuous lengths of belt laminates 406, 408, which are subsequently cut to form first and second elastic belts 106, 108 on diapers 100.

As shown in Figures 4, 5B, 5E1, and 5E2, the chassis 102 are transferred from the carrier apparatus 308 to a nip 316 between the carrier apparatus 308 and a roll 318 where the chassis 102 is combined with continuous lengths of advancing front belt 406 and back belt 408. The front belt laminate 406 and the back belt laminate material 408 each include a wearer facing surface 312 and an opposing garment facing surface 314. As such, the second surface 162b of the outer layer belt substrate 162 may define some or all the garment facing surface 314, and the second surface 164b of the inner layer belt substrate 164 may define some or all the wearer facing surface 312. The wearer facing surface 312 of the first belt laminate 406 may be combined with the garment facing surface 134 of the chassis 102 along the first waist region 116, and the wearer facing surface 312 of the second belt laminate 408 may be combined with the garment facing surface 134 of the chassis 102 along the second waist region 118. As shown in Figure 4, adhesive 320 may be intermittently applied to the wearer facing surface 312 of the first and second belt laminates 406, 408 before combining with the discrete chassis 102 at the nip 316 between roll 318 and the carrier apparatus 308.

Referring back to Figures 4, 5E1, and 5E2 a continuous length of absorbent articles 400 are defined by multiple discrete chassis 102 spaced from each other along the machine direction MD and connected with each other by the second belt laminate 408 and the first belt laminate 406. As shown in Figure 4, the continuous length of absorbent articles 400 advances from the edge transformation apparatus 331 to a folding apparatus 332. At the folding apparatus 332, each chassis 102 is folded in the cross direction CD parallel to or along a lateral axis 126 to place the first waist region 116, and specifically, the inner, body facing surface 132 into a facing, surface to surface orientation with the inner, body surface 132 of the second waist region 118. The folding of the chassis also positions the wearer facing surface 312 of the second belt laminate 408 extending between each chassis 102 in a facing relationship with the wearer facing surface 312 of the first belt laminate 406 extending between each chassis 102.

As shown in Figures 4 and 5F, the folded discrete chassis 102 connected with the first and second belt laminates 406, 408 are advanced from the folding apparatus 332 to a bonder apparatus 334. The bonder apparatus 334 operates to bond an overlap area 362, thus creating discrete bonds 336a, 336b. The overlap area 362 includes a portion of the second belt laminate 408 extending between each chassis 102 and a portion of the first belt laminate 406 extending between each chassis 102. It is to be appreciated that the bonder apparatus 334 may be configured in various ways to create bonds 336a, 336b in various ways, such as for example with heat, adhesives, pressure, and/or ultrasonics. It is also to be appreciated that in some embodiments, the apparatus 300 may also be configured to refastenably bond the overlap area 362, in addition to or as opposed to permanently bonding the overlap area 362. Thus, the discrete bonds 336a, 336b may be configured to be refastenable, such as with hooks and loops.

Referring now to Figures 4 and 5G, the continuous length of absorbent articles 400 are advanced from the bonder 334 to a cutting apparatus 338 where the first belt laminate 406 and the second belt laminate 408 are cut along the cross direction CD between adjacent bonds 336a, 336b to create discrete absorbent articles 100. As shown in Figure 5G, the first belt laminate 406 and the second belt laminate 408 are cut into discrete pieces to form the front and back elastic belts 106, 108, each having a pitch length, PL, extending along the machine direction MD. As such, bond 336a may correspond with and form a first side seam 178 on an absorbent article 100, and the bond 336b may correspond with and form a second side seam 180 on a subsequently advancing absorbent article.

It is to be appreciated that the methods and apparatuses herein may be configured to assemble absorbent articles with various components having various graphic designs. As previously mentioned, some embodiments of assembled diapers may include components that are combined during manufacture, wherein each component includes printed graphics. In particular, the graphics may include low print intensity zones positioned in areas where the components are combined. Thus, the low intensity zones may help reduce the noticeable results of imprecise placement of one printed component onto another printed component wherein the graphics on the separate components may otherwise appear disjointed and/or misaligned.

For example, the apparatus 300 discussed above with reference to Figure 4 may be configured to assemble diaper pants 100 with graphics printed on the front and/or back elastic belts 106, 108 as well as the chassis 102, such as shown in Figures 5GA and 5GA1. As discussed in more detail below with reference to Figures 5A1A-5E1A, the elastic laminates 406, 408 and one or more components of the chassis 102 may include graphics. During the assembly process, the chassis 102 and the elastic laminates 406, 408 may be assembled such that the respective graphics are aligned to provide the appearance of contiguous designs that extend across more than one of the assembled elastic belts 106, 108 and/or chassis 102. Thus, the chassis 102 and/or the elastic laminates 406, 408 may each include graphics with zones of relatively low print densities positioned in areas where the elastic laminates 406, 408 and chassis 102 are combined. In turn, the low intensity zones may help reduce the noticeable results of imprecise placement of the chassis 102 onto the elastic laminates 406, 408.

Figure 5A1A is a view of a continuous length of an advancing first substrate 162 from Figure 4 taken along line A1-A1 and showing a second embodiment of a graphic configuration. As shown in Figures 4 and 5A1A, the outer belt substrate 162 advances in the machine direction and may include graphics G printed on the first surface 162a of the outer layer belt substrate 162. As shown in Figure 5A1A, although the graphics G are printed on the first surface 162a of the outer layer belt substrate 162, the graphics G may be visible through the second surface 162b. It is also to be appreciated that the graphics G may be printed on either or both the first and second surfaces 162a, 162b of the outer belt substrate 162. It is also to be appreciated that graphics may be printed on either or both the first and second surfaces 164a, 164b of the inner belt substrate 164.

As shown in Figure 5A1A, the graphics G extend in the machine direction MD and includes a first low intensity zone ZLA, a second low intensity zone ZLB, a first high intensity zone ZH1, and a second high intensity zone ZH2. The low intensity zones ZLA, ZLB is are central zones positioned between the laterally opposing first high intensity zone ZH1 and the second high intensity zone ZH2. In addition, each graphic G defines a closed perimeter wherein the first low intensity zone ZLA is separated from the second low intensity zone ZLB in the machine direction MD, wherein the first and second high intensity zones ZH1, ZH2 are connected with and separated by the first low intensity zone ZLA and the second low intensity zone ZLB. For the purposes of clarity, dashed lines 401 are shown in Figure 5A1A to represent example boundaries between the high intensity zones ZH1, ZH2 and the low intensity zones ZLA, ZLB. It is to be appreciated that such boundaries between the high intensity zones ZH1, ZH2 and the low intensity zones ZLA, ZLB can also be curved, angled, and/or straight. As shown in Figure 5A1A, the low intensity zones ZLA, ZLB of the graphics G define a width, Wz, in the cross direction CD. It is to be appreciated that widths Wz of the low intensity zones ZLA, ZLB may vary. In some embodiments, the width Wz may be from about 8 mm to about 30 mm. In some embodiments, the width Wz may also be expressed in terms relative to the width W of the belt substrate 162 and/or the elastic laminate 402. For example, in some embodiments, the width W of the outer belt substrate 162 and/or the elastic laminate 402 may be about 8 to about 75 times the width Wz of the low intensity zones ZLA, ZLB. Although the low intensity zones ZLA, ZLB of the graphics G are depicted as extending contiguously in the cross direction CD between the high intensity zones ZH1, ZH2, it is to be appreciated that the low intensity zones ZLA, ZLB of the graphics G may be defined by discrete lengths extending in the cross direction CD. It is to also to be appreciated that the graphics G may be printed to have differing designs from each other along the machine direction MD and/or cross direction CD. With reference to Figure 5A1A, the central zones ZLA, ZLB of the graphic G may be positioned entirely within the central region 166c of the elastic laminate 402. As previously discussed, it is also to be appreciated the widths of the regions 166a, 166b, 166c may vary. For example, in some embodiments, the central region 166c may be about 33% of the width W of the elastic laminate 402. In some embodiments, the first and second outer longitudinal regions 166a, 166b and/or the central region may each be about 1/3 of the width W of the elastic laminate 402.

With continued reference to Figures 4 and 5BA, from the nip rolls 502 the continuous elastic laminate 402 advances in the machine direction MD to the cutter 506 that cuts the continuous elastic laminate 402 into two continuous elastic belt laminates, referred to as a first elastic belt laminate 406 and a second elastic belt laminate 408. In particular, the cutter 506 operates to cut the elastic laminate 402 along the machine direction and through the first and second low intensity zones ZLA, ZLB to form the first continuous elastic laminate 406 and the second continuous elastic laminate 408. As such, the cutter 506 also operates to divide the graphic G into a first graphic G1 and a second graphic G2, wherein the first graphic G1 is positioned on the first elastic laminate 406 and the second graphic G2 is positioned on the second elastic laminate 408. A first portion ZLA1 of the first central zone ZLA defines a first low intensity zone of the first graphic G1 and extends from the inner longitudinal edge 107b of the first continuous elastic laminate 406 to the first high intensity zone ZH1. And a first portion ZLB1 of the second central zone ZLB defines a second low intensity zone of the first graphic G1 and extends from the inner longitudinal edge 107b of the first continuous elastic laminate 406 to the first high intensity zone ZH1. In addition, a second portion ZLA2 of the first central zone ZLA defines a first low intensity zone of the second graphic G2 and extends from the inner longitudinal edge 109b of the second continuous elastic laminate 408 to the second high intensity zone ZH2. And a second portion ZLB2 of the second central zone ZLB defines a second low intensity zone of the second graphic G2 and extends from the inner longitudinal edge 109b of the second continuous elastic laminate 408 to the second high intensity zone ZH2. Thus, cutting the elastic laminate 402 in the machine direction MD through the low intensity zones ZLA, ZLB may help reduce noticeable visible results of an imprecise and/or crooked cut line that defines the inner longitudinal edges 107b, 109b of the first and second elastic laminates 406, 408.

As shown in Figure 5BA, the first belt laminate 406 extends between the outer longitudinal edge 107a and the inner longitudinal edge 107b to define a width W1 in the cross direction CD. And the second belt laminate 408 extends between the outer longitudinal edge 109a and the inner longitudinal edge 109b to define a width W2 in the cross direction CD. In addition, the low intensity zones ZLA1, ZLB1 of the first graphic G1 may define a width Wz1 in the cross direction CD, and the low intensity zones ZLA2, ZLB2 of the second graphic G2 may define a width Wz2 in the cross direction CD. It is to be appreciated that W2 may be greater than W1. It is also to be appreciated that in some configurations, W1 may be equal to or greater than W2. In some embodiments, the widths W1 and/or W2 may be from about 120 mm to about 300 mm. In addition, the widths Wz1, Wz2 of the low intensity zones ZLA1, ZLB1, ZLA2, ZLB2 may be expressed in terms relative to the width W1, W2 of the first and second belt laminates 406, 408, respectively. For example, in some embodiments, the widths W1, W2 of the first and/or second belt laminates 406, 408 may be about 8 to about 75 times the widths Wz1, Wz2 of the low intensity zones ZLA1, ZLB1, ZLA2, ZLB2 of graphics G1 and/or G2 as viewed from same side of the first and/or second belt laminates 406, 408.

As previously mentioned, the chassis 102 may also include graphics. For example, as shown in Figures 4 and 5CA, the continuous length of chassis assemblies 302 are advanced in a machine direction MD may include chassis graphics GC printed thereon. It is to be appreciated that the chassis graphics GC may be printed on various chassis components, such as the backsheet 136, and may be printed prior to or during assembly of the chassis components. In some configurations, the chassis graphics GC may be printed on a backsheet film layer that is subsequently covered by a nonwoven layer such that the chassis graphics are visible through the nonwoven layer. It is also to be appreciated that the various printing processes may be used to print the chassis graphics GC, such as for example, ink jet, flexography, and/or gravure printing processes as discussed above.

It is also to be appreciated that the chassis graphics GC may be configured in various different designs. For example, as shown in Figure 5CA, the chassis graphics GC may be configured as first and second stripes GC1, GC2 extending contiguously along the machine direction of the continuous length of chassis assemblies 302. It is to be appreciated that in some embodiments, the chassis graphics GC may be printed as discrete lengths separated from each other along the machine direction MD. With continued reference to Figure 5CA, the chassis graphics may include low intensity zones ZLC and high intensity zones ZHC, wherein lengths of the high intensity zones ZHC are separated from each other by the low intensity zones ZLC along the machine direction MD. As discussed above, the continuous length of chassis assemblies 302 may include absorbent assemblies 140 sandwiched between topsheet material 138 and backsheet material 136, leg elastics, barrier leg cuffs and the like.

With continued reference to Figures 4 and 5D1A, the continuous length of chassis assemblies 302 advance to the carrier apparatus 308 and are cut into discrete chassis 102 with knife roll 306, while advancing in the orientation shown in Figure 5D1A, wherein the longitudinal axis 124 of each chassis 102 is generally parallel with the machine direction MD. More particularly, the knife roll 306 operates to cut the continuous length of chassis assemblies 302 in the cross direction CD between high intensity zones ZHC. In some graphic embodiments wherein the low intensity zones extend contiguously between the high intensity zones ZHC, the knife roll 306 operates to cut the continuous length of chassis assemblies 302 in the cross direction CD through the low intensity zones ZLC. As shown in Figure 5D1A, the discrete chassis 102 may have a pitch length PLC extending between the first lateral end edge 144 and the second lateral end edge 146. In addition, the chassis 102 may also include graphics GC1, GC2 each having a first low intensity zone ZLC1 and a second low intensity zone ZLC2 separated from each other by a high intensity zone ZHC. The first low intensity zone ZLC1 may extend longitudinally from the high intensity zone ZHC toward the first lateral end edge 144 of the chassis 102. The second low intensity zone ZLC2 may extend longitudinally from the high intensity zone ZHC toward the second lateral end edge 146 of the chassis 102. In some embodiments, either or both the low intensity zones ZLC1, ZLC2 may extend contiguously all the way to end edges 144, 146. And in some embodiments, either or both the low intensity zones ZLC1 and/or ZLC2 may not extend completely to the end edges 144, 146.

For the purposes of clarity, dashed lines 401 are shown in Figure 5D1A to represent example boundaries between the high intensity zones ZHC and the low intensity zones ZLC1, ZLC2. It is to be appreciated that such boundaries between the high intensity zones ZH and the low intensity zones ZLC1, ZLC2 can also be curved, angled, and/or straight. As shown in Figure 5D1A, the first low intensity zones ZLC1 of the graphics GC1, GC2 may define a length, Lz1, in the machine direction MD, and the second low intensity zones ZLC2 of the graphics GC1, GC2 may define a length, Lz2, in the machine direction MD. It is to be appreciated that lengths Lz1, Lz2 of the low intensity zones ZLC1, ZLC2 may vary. In some embodiments, the lengths Lz1, Lz2 may be from about 4 mm to about 15 mm. In some embodiments, the lengths Lz1, Lz2 may also be expressed in terms relative to the pitch length PLC of the chassis 102. For example, in some embodiments, the pitch length PLC of the chassis 102 may be about 20 to about 150 times the lengths Lz1, Lz2 of either or both the low intensity zones ZLC1, ZLC2.

As discussed above with reference to Figure 4, after the discrete absorbent chassis 102 are cut by the knife roll 306, the carrier apparatus 308 rotates and advances the discrete chassis 102 in the machine direction MD in the orientation shown in Figure 5D1A. The carrier apparatus 308 also rotates while at the same time changing the orientation of the advancing chassis 102. In changing the chassis orientation, the carrier apparatus 308 may turn each chassis 102 such that the lateral axis 126 of the chassis 102 is parallel or generally parallel with the machine direction MD, such as shown in Figure 5D2A. As discussed below with reference to Figures 4, 5E1A, 5FA, 5GA, and 5G1A, the chassis 102 are transferred from the carrier apparatus 308 and combined with advancing, continuous lengths of belt laminates 406, 408, which are subsequently cut to form first and second elastic belts 106, 108 on diapers 100.

As shown in Figures 4, 5BA, and 5E1A, the chassis 102 are transferred from the carrier apparatus 308 to the nip 316 between the carrier apparatus 308 and the roll 318 where the chassis 102 is combined with continuous lengths of advancing front belt 406 and back belt 408. As shown in Figure 5E1A, each chassis 102 may be combined with the front belt 406 and back belt 408 such that the chassis graphics GC1, GC2 are aligned with the belt graphics G1, G2 to form a contiguous design. In particular, the first low intensity zones ZLC1 of the chassis graphics CG1 may be aligned with the low intensity zone ZLA1 of the first graphics G1, and the second low intensity zones ZLC2 of the chassis graphics CG1 may be aligned with the low intensity zone ZLA2 of the second graphics G2. In addition, the first low intensity zones ZLC1 of the chassis graphics CG2 may be aligned with the low intensity zone ZLB1 of the first graphics G1, and the second low intensity zones ZLC2 of the chassis graphics CG2 may be aligned with the low intensity zone ZLB2 of the second graphics G2. As such, the low intensity zones ZLA1, ZLB1 of graphics G1; the low intensity zones ZLA2, ZLB2 of graphics G2; and the low intensity zones ZLC1, ZLC2 of graphics GC1, GC2 are positioned in areas where the front belt 406, back belt 408, and chassis 102 are combined. Thus, the low intensity zones may help reduce the noticeable results of imprecise placement of the chassis 102 onto the front and/or back belts 406, 408, wherein the graphics on the chassis 102, front belt 406, and/or back belt 408 may otherwise appear disjointed and/or misaligned.

With continued reference to Figures 4 and 5E1A, the continuous length of absorbent articles 400 advances to the folding apparatus 332. At the folding apparatus 332, each chassis 102 is folded in the cross direction CD parallel to or along a lateral axis 126 to place the first waist region 116, and specifically, the inner, body facing surface 132 into a facing, surface to surface orientation with the inner, body surface 132 of the second waist region 118. The folding of the chassis also positions the wearer facing surface 312 of the second belt laminate 408 extending between each chassis 102 in a facing relationship with the wearer facing surface 312 of the first belt laminate 406 extending between each chassis 102.

As shown in Figures 4 and 5FA, the folded discrete chassis 102 connected with the first and second belt laminates 406, 408 are advanced from the folding apparatus 332 to a bonder apparatus 334. The bonder apparatus 334 operates to bond an overlap area 362, thus creating discrete bonds 336a, 336b. The overlap area 362 includes a portion of the second belt laminate 408 extending between each chassis 102 and a portion of the first belt laminate 406 extending between each chassis 102. It is to be appreciated that in some embodiments, the apparatus 300 may be configured to refastenably bond the overlap area 362, in addition to or as opposed to permanently bonding the overlap area 362. Thus, the discrete bonds 336a, 336b may be configured to be refastenable, such as with hooks and loops.

Referring now to Figures 4, 5GA, and 5GA1, the continuous length of absorbent articles 400 are advanced from the bonder 334 to a cutting apparatus 338 where the first belt laminate 406 and the second belt laminate 408 are cut along the cross direction CD between adjacent bonds 336a, 336b to create discrete absorbent articles 100. As shown in Figures 5GA and 5G1A, the first belt laminate 406 and the second belt laminate 408 are cut into discrete pieces to form the front and back elastic belts 106, 108, each having a pitch length, PL, extending along the machine direction MD. As such, bond 336a may correspond with and form a first side seam 178 on an absorbent article 100, and the bond 336b may correspond with and form a second side seam 180 on a subsequently advancing absorbent article. In addition, the graphics G1 and G2 may also be configured to align with each other at the side seams 178, 180 to provide the appearance of a contiguous design that extends across the side seams 178, 180.

It is to be appreciated that the processes and apparatuses herein may be configured to manufacture various types of diaper pants having the graphics G1, G2 discussed above. In some embodiments, the diaper pants 100 may include a chassis 102 and elastic belts 106, 108 configured in different ways other than as depicted in Figures 1A-2B. For example, Figures 6A-7 show a diaper pant 100 having many of the same components as described above with reference to Figures 1A-2B, except the outer layer 162 of the elastic belts 106, 108 is configured as a contiguous outer cover 161 that extends through the first waist region 116, crotch region 119, and second waist region 118. Thus, as shown in Figure 7, the outer cover 161 also includes a first waist end region 116, a crotch region 119, and an opposing second waist end region 118. The outer cover 161 also includes a garment facing surface 162b and an opposing wearer facing surface 162a. As such, elastic members 168 of the elastic belts 106, 108 may be connected with the wearer facing surface 162a of the outer cover 161. And the chassis 102 may be positioned on the wearer facing surface 162a of the outer cover 161. As such, the backsheet 136 may include a portion of the outer cover 161. In addition, the outer cover 161 may include a first longitudinal side edge 128a and a second longitudinal side edge 130a that are positioned laterally outboard the first longitudinal side edge 128 of the chassis 102 and second longitudinal side edge 130 of the chassis 102, respectively, as shown in Figure 7. As shown in Figures 6A and 7, the first longitudinal side edge 128a may define the perimeter 112a of one leg opening 112, and the second longitudinal side edge 130a may define the perimeter 112b of the other leg opening 112. It is to be appreciated also that the first longitudinal side edge 128a and a second longitudinal side edge 130a may aligned with or positioned laterally inboard of the first longitudinal side edge 128 of the chassis 102 and second longitudinal side edge 130 of the chassis 102, respectively. As such, in some embodiments, the perimeter 112a of one leg opening 112 may be defined by portions of the first longitudinal edges 128, 128a, and the perimeter 112b of the other leg opening may be defined by portions of the second longitudinal edges 130, 130a.

Figure 6B shows a front plan view of a diaper pant 100 in a laid flat condition illustrating various regions of the diaper pant 100. And 6C shows a rear plan view of the diaper pant 100 in a laid flat condition illustrating various regions of the diaper pant 100. As discussed above, the diaper pant 100 defines include an inner, body facing surface 132, and an outer, garment facing surface 134. The diaper pant 100 also includes a crotch end 190 that is defined by a lateral fold line 192 in the crotch region 119. As such, the lateral fold line 192 divides the crotch region into a first crotch region 119a and a second crotch region 119b.

The diaper pant 100 is shown in Figures 6A-6C as having a first elastic belt 106, and a second elastic belt 108. The first belt 106 has a first end region 106a, an opposing second end region 106b, and a central region 106c. And the second belt 108 has a first end region 108a, an opposing second end region 108b, and a central region 108c. The first end regions 106a, 108a are connected together at a first side seam 178, and the second end regions are 106b, 108b are connected together at a second side seam 180. As shown in Figures 6B and 6C, the distance between the first longitudinal side edge 111a and the second longitudinal side edge 111b defines the pitch length, PL, of the first elastic belt 106, and the distance between the first longitudinal side edge 113a and the second longitudinal side edge 113b defines the pitch length, PL, of the second elastic belt 108.

The first end region 106a the first belt 106 may extend approximately 20% to 40% of the pitch length PL of the diaper pant 100 in an assembled, laid-flat, relaxed condition, and the first end region 108a the second belt 108 may extend approximately 20% to 40% of the pitch length PL of the diaper pant 100 in an assembled, laid-flat, relaxed condition. The second end region 106b the first belt 106 may extend approximately 20% to 40% of the pitch length PL of the diaper pant 100 in an assembled, laid-flat, relaxed condition, and the second end region 108b the second belt 108 may extend approximately 20% to 40% of the pitch length of the diaper pant 100 in an assembled, laid-flat, relaxed condition. The central region 106c the first belt 106 may extend approximately 20% to 60% of the pitch length PL of the diaper pant 100 in an assembled, laid-flat, relaxed condition, and the central region 108c the second belt 108 may extend approximately 20% to 60% of the pitch length PL of the diaper pant 100 in an assembled, laid-flat, relaxed condition.

The diaper pant 100 in Figures 6B and 6C is also shown as having a longitudinal length LL that is defined by the distance between the first waist edge 121 and the crotch end 190 (or the lateral fold line 192), or if longer, the distance from the second waist edge 122 to the crotch end 190 (or the lateral fold line 192). The longitudinal length LL may be measured along the longitudinal centerline 124 of the diaper pant 100. As shown in Figures 6B-6C, the first waist region 116 extends a distance generally in the longitudinal direction from the waist edge 121 along the side seams 178, 180 to the leg openings 112, and the second waist region 118 extends a distance generally in the longitudinal direction from the waist edge 122 along the side seams 178, 180 to the leg openings 112. Hence, a first crotch region 119a extends a distance from the crotch end 190 to the first waist region 116, and a second crotch region 119b extends a distance from the crotch end 190 to the second waist region 118. In some embodiments, the first waist region 116 and/or the second waist region 118 may extend about two-thirds the longitudinal length LL of the assembled diaper pant 100. In addition, the first crotch region 119a and/or the second crotch region 119b may extend about one-third the longitudinal length LL of the assembled diaper pant 100.

The diaper pant 100 shown in Figures 6A-6C also includes printed graphics G1, G2 on the first elastic belt 106 and the second elastic belt 108 wherein the first graphic G1 includes a high intensity zone ZH1 and a low intensity zone ZL1. And the second graphic G2 includes a high intensity zone ZH2 and a low intensity zone ZL2. As shown in Figure 6B, the low intensity zone ZL1 is positioned along the first and second longitudinal side edges 111a, 111b and the perimeters 112a, 112b of the leg openings 112 in the front waist region 116 and the first crotch region 119a. As shown in Figure 6C, the low intensity zone ZL2 is positioned along the first and second longitudinal side edges 113a, 113b and the perimeters 112a, 112b of the leg openings 112 in the back waist region 118 and the second crotch region 119b. In addition, the high intensity zones ZH1, ZH2 are positioned away from the perimeters 112a, 112b of the leg openings. As previously discussed, the low intensity zones ZL1, ZL2 are positioned in regions of the diapers 100 that may be subject to various cutting and/or folding transformations during the assembly process so as to reduce noticeable visible results of imprecisions and/or inconsistencies of such transformations.

With continued reference to Figures 6B-6C, the low intensity zone ZL1 of the graphic G1 on the front belt 106 is positioned between the high intensity zone ZH1, the crotch end 190, and perimeters 112a, 112b of the leg openings 112. And the low intensity zone ZL2 of the graphic G2 on the second belt 108 is positioned between the high intensity zone ZH2, the crotch end 190, and perimeters 112a, 112b of the leg openings 112. For the purposes of clarity, dashed lines 401 are shown in Figures 6B-6C to represent example boundaries between the high intensity zones ZH1, ZH2 and the low intensity zones ZL1, ZL2. It is to be appreciated that such boundaries between the high intensity zones ZH1, ZH2 and the low intensity zones ZL1, ZL2 can also be curved, angled, and/or straight. As shown in Figures 6B-6C, the low intensity zone ZL1 of the graphic G1 on the front belt 106 may extend from the high intensity zone ZH1 entirely to the perimeter edges 112a, 112b, and the low intensity zone ZL2 of the graphic G2 on the back belt 108 may extend from the high intensity zone ZH entirely to the perimeter edges 112a, 112b. It is to be appreciated that in some embodiments, the low intensity zones ZL1, ZL2 may or may not extend all the way to the crotch end 190. As also shown in Figures 6B-6C, the low intensity zone ZL1 of the graphic G1 on the front belts 106 may extend contiguously from the first longitudinal side edge 111a to the second longitudinal side edge 111b, and the low intensity zone ZL2 of the graphic G2 on the back belt 108 may extend contiguously from the first longitudinal side edge 113a to the second longitudinal side edge 113b. It is to be appreciated that in some embodiments, the low intensity zones ZL1, ZL2 may not extend all the way to one of or both of the longitudinal side edges 111a, 111b on the front belt 106 and/or all the way to one of or both of the longitudinal side edges 113a, 113b on the back belt 108. It is to be appreciated that in some embodiments, the low intensity zones of printed graphics could be arranged to partially or completely surround the perimeters 112a, 112b of the leg openings 112.

As previously discussed, the low intensity zones ZL1, ZL2 are positioned in regions of the diapers 100 that may be subject to various cutting and/or folding transformations during the assembly process so as to reduce noticeable visible results of imprecisions and/or inconsistencies of such transformations. Thus, it is also to be appreciated that the low intensity zones ZL1, ZL2 discussed herein may be devoid of additional graphics. As such, it may be desirable in some embodiments to manufacture absorbent articles with graphics having a high intensity zone and a low intensity zone wherein the low intensity zone is devoid of any other printed graphics or the like.

As shown in Figures 6B-6C, the low intensity zone ZL1 of the first graphic G1 may also define a width Wz1, and the low intensity zone ZL2 of the second graphic G2 may define a width Wz2. It is to be appreciated that widths Wz1, Wz2 of the low intensity zones ZL1, ZL2 may vary. In some embodiments, the widths Wz1, Wz2 may be from about 4 mm to about 15 mm. In some embodiments, the widths Wz1, Wz2 may be expressed in terms relative to the longitudinal length LL of the assembled diaper pant 100. For example, in some embodiments, the longitudinal length LL of the assembled diaper pant 100 may be about 10 to about 125 times the widths Wz1, Wz2 of graphics G1 and/or G2.

As discussed above, substrates and/or components that may be incorporated into manufactured absorbent articles, such as shown in Figures 6A-7, may include graphics positioned and/or printed in such a manner so as to reduce noticeable visible results of imprecise and/or inconsistent manufacturing operations performed in areas where the printing is located. And Figure 8 shows a converting apparatus 300 configured to assemble diaper pants such as shown in Figures 6A-7. As shown in Figure 8, a first continuous substrate layer in the form of a continuous length of outer layer belt substrate 162 is combined with first and second separate continuous lengths of inner layer belt substrates 164', 164" and elastics 168 form a continuous elastic laminate 402. The outer layer belt substrate 162 also defines the outer cover 161 discussed above with reference to Figures 6A-7. With reference to Figures 8, 9A1, 9A2, and 9B, continuous lengths of outer layer belt substrate 162, first and second inner layers of belt substrate 164', 164", outer elastic strands 170 and inner elastic strands 172 are advanced in a machine direction MD and combined at nip rolls 502 to form the continuous elastic laminate 402.

Before entering the nip rolls 502, the outer layer belt substrate 162 and/or the first and second inner belt substrates 164', 164" may be printed with graphics having high intensity zones and low intensity zones as discussed above. It is to be appreciated that the graphic printing may be done during the assembly process and/or may done separate to the assembly process, such as for example, printing the substrates off line where the printed substrates may be stored until needed for production.

As shown in Figures 8, 9A1, and 9A2, the outer belt substrate 162 includes first surface 162a and an opposing second surface 162b, and defines a width W in the cross direction between opposing longitudinal edges 163a, 163b. The first inner belt substrate 164' includes first surface 164a and an opposing second surface 164b, and defines a width in the cross direction CD between opposing first and second longitudinal edges 165a, 165b. And the second inner belt substrate 164" includes first surface 164a and an opposing second surface 164b, and defines a width in the cross direction CD between opposing first and second longitudinal edges 165a, 165b. As shown in Figure 9A2, the width W of the outer belt substrate 162 may be greater than the widths of the inner belt substrates 164', 164". And the width W of the outer belt substrate 162 may also define the width W of the elastic laminate 402.

As shown in Figures 8 and 9A1, the outer belt substrate 162 advances in the machine direction and may include graphics G1, G2 printed on the first surface 162a of the outer layer belt substrate 162. As shown in Figure 9A1, although the graphics G1, G2 are printed on the first surface 162a of the outer layer belt substrate 162, the graphics G1, G2 may be visible through the second surface 162b. It is also to be appreciated that the graphics G1, G2 may be printed on either or both the first and second surfaces 162a, 162b of the outer belt substrate 162. It is also to be appreciated that graphics may be printed on either or both the first and second surfaces 164a, 164b of the first and second inner belt substrates 164', 164".

As shown in Figure 9A1, each graphic G1, G2 extends in the machine direction MD. The first graphic G1 includes a low intensity zone ZL1 and a high intensity zone ZH1. And the second graphic G2 includes a low intensity zone ZL2 and a high intensity zone ZH2. The low intensity zones ZL1, ZL2 are central zones positioned between the laterally opposing high intensity zones ZH1, ZH2. For the purposes of clarity, dashed lines 401 are shown in Figure 9A1 to represent example boundaries between the high intensity zones ZH1, ZH2 and the low intensity zones ZL1, ZL2. It is to be appreciated that such boundaries between the high intensity zones ZH1, ZH2 and the low intensity zones ZL1, ZL2 can also be curved, angled, and/or straight. It is also to be appreciated that some graphics may be configured with a single low intensity zone that extends in the cross direction CD contiguously between the high intensity zones ZH1, ZH2, such as discussed above with reference to Figure 5A1.

As shown in Figure 9A1, the low intensity zone ZL1 of the first graphic G1 defines a width, Wz1, in the cross direction CD. And the low intensity zone ZL2 of the first graphic G2 defines a width, Wz2, in the cross direction CD. It is to be appreciated that widths Wz1, Wz2 of the low intensity zones ZL1, ZL2 may vary. In some embodiments, the widths Wz1, Wz2 may be from about 4 mm to about 15 mm. In some embodiments, the width W of the belt substrate 162 and/or elastic laminate 402 may be from about 240 mm to about 600 mm. In some embodiments, the widths Wz1, Wz2 may also be expressed in terms relative to the width W of the belt substrate 162 and/or the elastic laminate 402. For example, in some embodiments, the width W of the outer belt substrate 162 and/or the elastic laminate 402 may be about 8 to about 150 times the widths Wz1, Wz2 of the low intensity zones ZL1, ZL2. Although the low intensity zones ZL1, ZL2 are depicted as extending contiguously in the machine direction MD, it is to be appreciated that either or both the low intensity zones ZL1, ZL2 may be defined by discrete lengths extending in the machine direction MD. It is to also to be appreciated that the graphics G1, G2 may be printed to have differing designs from each other along the machine direction MD and/or cross direction CD. Also shown in Figure 9A1, the outer belt substrate 162, and thus the elastic laminate 402, may include first and second outer longitudinal regions 166a, 166b separated in the cross direction CD by a central region 166c. And either or both the low intensity zones ZL1, Zl2 of the graphics G1, G2 may be positioned entirely within the central region 166c of the elastic laminate 402. It is to be appreciated the widths of the regions 166a, 166b, 166c may vary. For example, in some embodiments, the central region 166c may be about 33% of the width W of the elastic laminate 402. In some embodiments, the first and second outer longitudinal regions 166a, 166b and/or the central region may each be about 1/3 of the width W of the elastic laminate 402.

With continued reference to Figure 8, before entering the nip rolls 502, the outer elastic strands 170 and inner elastic strands 172 are stretched in the machine direction MD. In addition, adhesive 504 may applied to the elastic strands 170, 172 as well as either or both of the continuous lengths of outer layer belt substrate 162 and inner layer belt substrates 164', 164" before entering nip rolls 502. As such, the elastic strands 168 are bonded between the first surface 162a of the outer layer belt substrate 162 and the first surfaces 164a of inner layer belt substrates 164', 164" at the nip rolls 502. Further, adhesive 504 may be applied intermittently along the lengths of the inner elastic strands 172 and/or intermittently along the length of either or both of the continuous lengths of outer layer belt substrate 162 and inner layer belt substrates 164', 164" before entering nip rolls 502. As previously discussed, the inner elastic strands 172 may be intermittently bonded to either or both of the continuous lengths of outer layer belt substrate 162 and inner layer belt substrates 164', 164" along the machine direction MD.

As shown in Figures 8 and 9A2, the continuous elastic laminate 402 includes a first elastic belt laminate 406 and a second elastic belt laminate 408. More particularly, the combination of the outer layer belt substrate 162, the first inner layer of belt substrate 164', and elastic strands 168 defines the first belt laminate 406. And the combination of the outer layer belt substrate 162, the second inner layer of belt substrate 164", and elastic strands 168 defines the second belt laminate 408. The first belt laminate 406 includes an outer longitudinal edge 163a and an inner longitudinal edge 107b that may define a substantially constant width, W1, in the cross direction CD. The inner longitudinal edge 107b may be defined by the second longitudinal edge 165b of the first inner belt substrate 164'. The second belt laminate 408 includes an outer longitudinal edge 163b and an inner longitudinal edge 109b that may define a substantially constant width, W2, in the cross direction CD. The inner longitudinal edge 109b may be defined by the second longitudinal edge 165b of the second inner belt substrate 164". In some configurations, W2 equal to W1. It is also to be appreciated that in some configurations, W1 may be less than or greater than W2. The first belt laminate 406 is separated in the cross direction from the second belt laminate 408 to define a gap between the inner longitudinal edge 107b of the first belt laminate 406 and the inner longitudinal edge 109b of the second belt laminate 408.

With continued reference to Figure 8, from the nip rolls 502 the continuous elastic laminate 402 advances in the machine direction MD to a cutter 507 that removes material from a central region of the continuous elastic laminate 402 to form holes 115 defined by perimeter edges 112c, such as shown in Figure 9B. The holes 115 are discrete and may be spaced apart from each other along the machine direction MD. The perimeter edges 112c may define all or portions of the perimeters 112a, 112b of the leg openings 112 mentioned above and shown in Figure 6A. As shown in Figure 9B, the cutter 507 may be configured to the holes 115 such that the perimeter edges 112c extend through either or both the low intensity zones ZL1, ZL2. Thus, cutting the holes 115 such that the perimeter edges 112c extend through the low intensity zones ZL1, ZL2 may help reduce noticeable visible results of an imprecise and/or crooked cut lines that define the leg openings 112.

It is to be appreciated that the cutter 507 may be configured to remove material from only the outer layer belt substrate 162. In some configurations, the cutter 507 may be configured to remove material from the outer belt substrate 162 as well as the first inner layer belt substrate 164' and/or second inner layer belt substrate 164". The cutter 507 may also be configured as a perforator that perforates the belt material with a line of weakness and wherein the belt material is separated along the line of weakness in a later step. It is also to be appreciated that the cutter 507 may be configured to form holes 115 in the continuous elastic laminate 402 before or after the continuous elastic laminate 402 is combined with the chassis 102.

As discussed above with reference to Figures 4, 5C, 5D1, and 5D2, and as shown in Figure 8, a continuous length of chassis assemblies 302 are advanced in a machine direction MD to a carrier apparatus 308 and are cut into discrete chassis 102 with knife roll 306, while advancing in the orientation shown in Figure 5D1. After the discrete absorbent chassis 102 are cut by the knife roll 306, the carrier apparatus 308 rotates and advances the discrete chassis 102 in the machine direction MD in the orientation shown in Figure 5D1. The carrier apparatus 308 also rotates while at the same time changing the orientation of the advancing chassis 102. In changing the chassis orientation, the carrier apparatus 308 may turn each chassis 102 such that the lateral axis 126 of the chassis 102 is parallel or generally parallel with the machine direction MD, such as shown in Figure 5D2.

As shown in Figures 8, 9E1, and 9E2, the chassis 102 are transferred from the carrier apparatus 308 to a nip 316 between the carrier apparatus 308 and a roll 318 where the chassis 102 is combined with the continuous elastic laminate 402. The chassis 102 may be spaced apart from each other along the machine direction MD on the continuous elastic laminate 402, wherein at least one hole 115 is positioned between two consecutive chassis 102. The continuous elastic laminate 402 includes a wearer facing surface 312 and an opposing garment facing surface 314. As such, the second surface 162b of the outer layer belt substrate 162 may define the garment facing surface 314. And the first surface 162a of the outer layer belt substrate 162 and the second surfaces 164b of the inner layer belt substrates 164', 164" may define the wearer facing surface 312. The wearer facing surface 312 of the continuous elastic laminate 402 may be combined with the garment facing surface 134 of the chassis 102. As shown in Figure 8, adhesive 320 may be intermittently applied to the wearer facing surface 312 of the continuous elastic laminate 402 before combining with the discrete chassis 102 at the nip 316 between roll 318 and the carrier apparatus 308.

With continued reference to Figures 8, 9E1, and 9E2, a continuous length of absorbent articles 400 are defined by multiple discrete chassis 102 spaced from each other along the machine direction MD and connected with each other by the continuous elastic laminate 402. As shown in Figure 8, the continuous length of absorbent articles 400 advances from the nip 316 to a folding apparatus 332. At the folding apparatus 332, the continuous elastic laminate 402 and each chassis 102 are folded in the cross direction CD parallel to or along a lateral axis 126 to place the first waist region 116, and specifically, the inner, body facing surface 132 into a facing, surface to surface orientation with the inner, body surface 132 of the second waist region 118. The folding operation creates the lateral fold line 192 that defines the crotch end 190 discussed above with reference to Figures 6B and 6C. The folding of the chassis also positions the wearer facing surface 312 of the second belt laminate 408 extending between each chassis 102 in a facing relationship with the wearer facing surface 312 of the first belt laminate 406 extending between each chassis 102.

As shown in Figures 8 and 9F, the folded continuous length of absorbent articles 400 are advanced from the folding apparatus 332 to a bonder apparatus 334. The bonder apparatus 334 operates to bond an overlap area 362, thus creating discrete bonds 336a, 336b. The overlap area 362 includes a portion of the second belt laminate 408 extending between each chassis 102 and a portion of the first belt laminate 406 extending between each chassis 102. As shown in Figure 9F, the discrete bonds 336a, 336b are positioned may extend through each graphic G1, G2. It is to be appreciated that the bonder apparatus 334 may be configured in various ways to create bonds 336a, 336b in various ways, such as for example with heat, adhesives, pressure, and/or ultrasonics. It is also to be appreciated that in some embodiments, the apparatus 300 may be configured to refastenably bond the overlap area 362, in addition to or as opposed to permanently bonding the overlap area 362. Thus, the discrete bonds 336a, 336b may be configured to be refastenable, such as with hooks and loops.

Referring now to Figures 8 and 9G, the continuous length of absorbent articles 400 are advanced from the bonder 334 to a cutting apparatus 338 where the first belt laminate 406 and the second belt laminate 408 are cut along the cross direction CD between adjacent bonds 336a, 336b to create discrete absorbent articles 100. As shown in Figure 9G, the continuous length of absorbent articles 400 are cut into discrete pieces to form the front and back elastic belts 106, 108, each having a pitch length, PL, extending along the machine direction MD and longitudinal length LL extending in the cross direction CD. As such, bond 336a may correspond with and form a first side seam 178 on an absorbent article 100, and the bond 336b may correspond with and form a second side seam 180 on a subsequently advancing absorbent article.

### Method for Measuring Print Color and Print Density

Print color and density on a printed nonwoven or film is measured using a hand held, 45°/0° configuration, hemispherical geometry spectrophotometer, the X-rite eXact Spectrophotometer (available from X-Ritc, Grand Rapids MI), or equivalent instrument, with a 4.0 mm optical aperture. This instrument measures print density based on reflection density expressed as the logarithm of the reciprocal of the reflectance factor. Set the scale to L*a*b* units, 2° Observer, C Illumination, Abs White Base, no Physical Filter, and the Density Standard of ANSI T. Measurements are performed in an environment controlled lab held at about 23 °C ± 2 C° and 50 % ± 2 % relative humidity.

Calibrate the instrument per the vender's instructions using the standard white board (available as PG2000 from Sun Chcmical-Vivitck Division, Charlotte, NC) cach day before analyses are performed. Remove the substrate to be measured from the sample article. If necessary, a cryogenic freeze-spray (e.g., Cyto-freeze, available from Control Company, Houston TX) can be used to facilitate removal. Samples are conditioned at about 23 °C ± 2 C° and 50 % ± 2 % relative humidity for 2 hours before testing.

Place the Standard White Board on a horizontal bench, standard side facing upward. Place the specimen flat on top of the Standard White Board with the printed side facing upward. Place the cXact spectrophotometer on the specimen such that the measurement site is free of folds and wrinkles and 100% of the measurement site is within the instrument's aperture. Take a reading for density and L*a*b* color and record each to the nearest 0.01 units.

In like fashion the measure is repeated on corresponding sites on five (5) substantially similar printed substrates and the density and L*a*b* color values averaged separately and reported to the nearest 0.01 units.

It is to be appreciated that the methods of assembly of diaper pants specifically described herein and illustrated in the accompanying drawings are non-limiting example embodiments. The features illustrated or described in connection with one non-limiting embodiment may be combined with the features of other non-limiting embodiments. Such modifications and variations are intended to be included within the scope of the present disclosure.

## Claims

1. A method for assembling disposable diaper pants, each diaper pant comprising a chassis (102) having a first end region (116) and an opposing second end region (118) separated from each other by a central region (119), and having a longitudinal axis (124) and a lateral axis (126), the chassis (102) comprising: a topsheet (138), a backsheet (136), and an absorbent core (140) disposed between the topsheet (138) and the backsheet (136), the method comprising the steps of:
advancing a continuous elastic laminate (402) in a machine direction, the elastic laminate (402) comprising a first longitudinal edge (163 a) and a second longitudinal edge (163b) defining a width, W, in a cross direction, the elastic laminate (402) further comprising a graphic (G), the graphic (G) extending in the machine direction and the cross direction and
comprising a central zone (ZL) positioned between laterally opposing first and second zones (ZH1, ZH2), wherein each zone comprises a maximum print density, wherein the maximum print density of the central zone (ZL) is less than or equal to 30% of the maximum print densities of the first and second zones (ZH1, ZH2), and wherein the central zone (ZL) defines a width, Wz, in the cross direction of less than or equal to about 10% of the width, W, of the elastic laminate (402);
cutting the elastic laminate (402) along the machine direction and through the central zone (ZL) to form a first continuous elastic laminate (406) and a second continuous elastic laminate (408), wherein the first and second continuous elastic laminates (406, 408) each include an inner longitudinal edge (107b, 109b) and an outer longitudinal edge (107a, 109a), and wherein a first portion (ZL1) of the central zone (ZL) extends along inner longitudinal edge (107b) of the first continuous elastic laminate (406) and a second portion (ZL2) of the central zone (ZL) extends along the inner longitudinal edge (109b) of the second continuous elastic laminate (408);
separating the first continuous elastic laminate (406) in the cross direction from the second continuous elastic laminate (408) to define a gap between the inner longitudinal edge (107b) of the first continuous elastic laminate (406) and the inner longitudinal edge (109b) of the second continuous elastic laminate (408); depositing a plurality of chassis (102) spaced apart from each other along the machine direction across the gap and onto the first continuous elastic laminate (406) and the second continuous elastic laminate (408); folding each chassis (102) along the lateral axis (126) to position the first continuous elastic laminate (406) into a facing relationship with the second continuous elastic laminate (408); and
cutting the first and second continuous elastic laminates (406, 408) in the cross direction to form discrete diaper pants (100).

2. The method of claim 1, wherein the width, W, is 240 mm to 600 mm.

3. The method according to any of the preceding claims, wherein the maximum print density of the central zone (ZL) is 0.15.

4. The method according to any of the preceding claims, wherein the step of cutting the first and second continuous elastic laminates (406, 408) further comprises cutting through the first and second zones (ZH1, ZH2).

5. The method according to any of the preceding claims, wherein the step of cutting the first and second continuous elastic laminates (406, 408) further comprises cutting the first and second continuous elastic laminates (406, 408) into discrete pieces having a pitch length, PL, extending along the machine direction, wherein the first zone (ZH1) extends continuously in the machine direction for less than or equal to 40% of the pitch length, PL.

6. The method according to any of the preceding claims, wherein the central zone (ZL) of the graphic (G) extends continuously in the machine direction.

7. The method according to any of the preceding claims, wherein the step of folding the chassis (102) along the lateral axis (126) further comprises aligning with the first zone (ZH1) and the second zone (ZH2) such that the first and second zones form a contiguous design on at least one diaper pant (100).

8. The method according to any of the preceding claims, wherein the elastic laminate (402) comprises first and second outer longitudinal regions (166a, 166b) separated in the cross direction by a central region (166c), wherein the central region (166c) is 33% of the width W of the elastic laminate (402), and wherein the central zone (ZL) of the graphic (G) is positioned in the central region (166c) of the elastic laminate (402).

9. The method according to any of the preceding claims, wherein the graphic (G) defines a closed perimeter wherein the central zone (ZL) comprises a first central zone (ZLA) separated from a second central zone (ZLB) in the machine direction, wherein the first and second zones (ZH1, ZH2) are connected with and separated by the first central zone (ZLA) and the second central zone (ZLB).

10. The method of claim 9, wherein the step of cutting the elastic laminate (402) along the machine direction further comprises cutting through the first and second central zones (ZLA, ZLB).

## Patentansprüche

1. Verfahren zum Zusammensetzen von Einwegwindelhöschen, wobei jedes Windelhöschen eine Grundeinheit (102) mit einem ersten Endbereich (116) und einem gegenüberliegenden zweiten Endbereich (118) umfasst, die voneinander durch einen Mittelbereich (119) getrennt sind, und eine Längsachse (124) und eine Querachse (126) aufweist, wobei die Grundeinheit (102) Folgendes umfasst:
eine Oberschicht (138), eine Unterschicht (136) und einen Absorptionskern (140), der zwischen der Oberschicht (138) und der Unterschicht (136) angeordnet ist,
wobei das Verfahren die folgenden Schritte umfasst:
Vorschieben eines ununterbrochenen elastischen Laminats (402) in einer Maschinenrichtung, wobei das elastische Laminat (402) eine erste Längskante (163a) und eine zweite Längskante (163b) umfasst, die eine Breite, W, in einer Querrichtung definieren, wobei das elastische Laminat (402) ferner eine grafische Darstellung (G) umfasst, wobei sich die grafische Darstellung (G) in der Maschinenrichtung und der Querrichtung erstreckt und eine Mittelzone (ZL) umfasst, die zwischen einander seitlich gegenüberliegenden ersten und zweiten Zonen (ZH1, ZH2) angeordnet ist, wobei jede Zone eine maximale Druckdichte umfasst, wobei die maximale Druckdichte der Mittelzone (ZL) kleiner als oder gleich 30 % der maximalen Druckdichten der ersten und der zweiten Zone (ZH1, ZH2) ist, und wobei die Mittelzone (ZL) eine Breite, Wz, in der Querrichtung von kleiner als oder gleich etwa 10 % der Breite, W, des elastischen Laminats (402) definiert;
Schneiden des elastischen Laminats (402) entlang der Maschinenrichtung und durch die Mittelzone (ZL), um ein erstes ununterbrochenes elastisches Laminat (406) und ein zweites ununterbrochenes elastisches Laminat (408) zu bilden, wobei das erste und das zweite ununterbrochene elastische Laminat (406, 408) jeweils eine innere Längskante (107b, 109b) und eine äußere Längskante (107a, 109a) einschließen, und wobei sich ein erster Abschnitt (ZL1) der Mittelzone (ZL) entlang der inneren Längskante (107b) des ersten ununterbrochenen elastischen Laminats (406) erstreckt und sich ein zweiter Abschnitt (ZL2) der Mittelzone (ZL) entlang der inneren Längskante (109b) des zweiten ununterbrochenen elastischen Laminats (408) erstreckt;
Trennen des ersten ununterbrochenen elastischen Laminats (406) in der Querrichtung von dem zweiten ununterbrochenen elastischen Laminat (408), um einen Spalt zwischen der inneren Längskante (107b) des ersten ununterbrochenen elastischen Laminats (406) und der inneren Längskante (109b) des zweiten ununterbrochenen elastischen Laminats (408) zu definieren; Aufbringen einer Vielzahl von Grundeinheiten (102), die entlang der Maschinenrichtung voneinander beabstandet sind, über den Spalt und auf das erste ununterbrochene elastische Laminat (406) und das zweite ununterbrochene elastische Laminat (408); Falten jeder Grundeinheit (102) entlang der Querachse (126), um das erste ununterbrochene elastische Laminat (406) in einer gegenüberliegenden Beziehung zum zweiten ununterbrochenen Laminat (408) anzuordnen; und
Schneiden des ersten und des zweiten ununterbrochenen elastischen Laminats (406, 408) in der Querrichtung, um separate Windelhöschen zu bilden (100).

2. Verfahren nach Anspruch 1, wobei die Breite, W, 240 mm bis 600 mm beträgt.

3. Verfahren nach einem der vorstehenden Ansprüche, wobei die maximale Druckdichte der Mittelzone (ZL) 0,15 beträgt.

4. Verfahren nach einem der vorstehenden Ansprüche, wobei der Schritt des Schneidens des ersten und des zweiten ununterbrochenen elastischen Laminats (406, 408) ferner das Schneiden durch die erste und die zweite Zone (ZH1, ZH2) umfasst.

5. Verfahren nach einem der vorstehenden Ansprüche, wobei der Schritt des Schneidens des ersten und des zweiten ununterbrochenen elastischen Laminats (406, 408) ferner das Schneiden des ersten und des zweiten ununterbrochenen elastischen Laminats (406, 408) in separate Teile mit einer Abstandslänge, PL, umfasst, die sich entlang der Maschinenrichtung erstreckt, wobei sich die erste Zone (ZH1) für kleiner als oder gleich 40 % der Abstandslänge, PL, ununterbrochen in der Maschinenrichtung erstreckt.

6. Verfahren nach einem der vorstehenden Ansprüche, wobei sich die Mittelzone (ZL) der grafischen Darstellung (G) ununterbrochen in der Maschinenrichtung erstreckt.

7. Verfahren nach einem der vorstehenden Ansprüche, wobei der Schritt des Faltens der Grundeinheit (102) entlang der Querachse (126) ferner ein Ausrichten an der ersten Zone (ZH1) und der zweiten Zone (ZH2) derart umfasst, dass die erste und die zweite Zone eine zusammenhängende Gestaltung an mindestens einem Windelhöschen (100) bilden.

8. Verfahren nach einem der vorstehenden Ansprüche, wobei das elastische Laminat (402) einen ersten und einen zweiten äußeren Längsbereich (166a, 166b) umfasst, die in der Querrichtung durch einen Mittelbereich (166c) getrennt sind, wobei der Mittelbereich (166c) 33 % der Breite, W, des elastischen Laminats (402) beträgt, und wobei die Mittelzone (ZL) der grafischen Darstellung (G) in dem Mittelbereich (166c) des elastischen Laminats (402) angeordnet ist.

9. Verfahren nach einem der vorstehenden Ansprüche, wobei die grafische Darstellung (G) einen geschlossenen Umfang definiert, wobei die Mittelzone (ZL) eine von einer zweiten Mittelzone (ZLB) in der Maschinenrichtung getrennte erste Mittelzone (ZLA) umfasst, wobei die erste und die zweite Zone (ZH1, ZH2) mit der ersten Mittelzone (ZLA) und der zweiten Mittelzone (ZLB) verbunden und durch diese getrennt werden.

10. Verfahren von Anspruch 9, wobei der Schritt des Schneidens des elastischen Laminats (402) entlang der Maschinenrichtung ferner das Schneiden durch die erste und die zweite Mittelzone (ZLA, ZLB) umfasst.

## Revendications

1. Procédé d'assemblage de couches-culottes jetables, chaque couche-culotte comprenant un châssis (102) ayant une première région d'extrémité (116) et une deuxième région d'extrémité opposée (118), séparées l'une de l'autre par une région centrale (119), et ayant un axe longitudinal (124) et un axe latéral (126), le châssis (102) comprenant : une feuille de dessus (138), une feuille de fond (136) et une âme absorbante (140) disposée entre la feuille de dessus (138) et la feuille de fond (136), le procédé comprenant les étapes consistant à :
faire avancer un stratifié élastique continu (402) dans un sens machine, le stratifié élastique (402) comprenant un premier bord longitudinal (163a) et un deuxième bord longitudinal (163b) définissant une largeur, W, dans une direction croisée, le stratifié élastique (402) comprenant en outre un graphisme (G), le graphisme (G) s'étendant dans le sens machine et la direction croisée et comprenant une zone centrale (ZL) positionnée entre des première et deuxième zones latéralement opposées (ZH1, ZH2), chaque zone comprenant une densité d'impression maximale, la densité d'impression maximale de la zone centrale (ZL) étant inférieure ou égale à 30 % des densités d'impression maximales des première et deuxième zones (ZH1, ZH2), et la zone centrale (ZL) définissant une largeur, Wz, dans la direction croisée inférieure ou égale à environ 10 % de la largeur, W, du stratifié élastique (402) ;
couper le stratifié élastique (402) le long du sens machine et à travers la zone centrale (ZL) pour former un premier stratifié élastique continu (406) et un deuxième stratifié élastique continu (408), les premier et deuxième stratifiés élastiques continus (406, 408) incluant chacun un bord longitudinal interne (107b, 109b) et un bord longitudinal externe (107a, 109a), et une première partie (ZL1) de la zone centrale (ZL) s'étendant le long du bord longitudinal interne (107b) du premier stratifié élastique continu (406) et une deuxième partie (ZL2) de la zone centrale (ZL) s'étendant le long du bord longitudinal interne (109b) du deuxième stratifié élastique continu (408) ;
séparer le premier stratifié élastique continu (406) dans la direction croisée par rapport au deuxième stratifié élastique continu (408) pour définir un espace entre le bord longitudinal interne (107b) du premier stratifié élastique continu (406) et le bord longitudinal interne (109b) du deuxième stratifié élastique continu (408) ; déposer une pluralité de châssis (102) espacés les uns des autres le long du sens machine à travers l'espace et sur le premier stratifié élastique continu (406) et le deuxième stratifié élastique continu (408) ; plier chaque châssis (102) le long de l'axe latéral (126) pour positionner le premier stratifié élastique continu (406) en une relation faisant face avec le deuxième stratifié élastique continu (408) ; et
couper les premier et deuxième stratifiés élastiques continus (406, 408) dans la direction croisée pour former des couches-culottes distinctes (100).

2. Procédé selon la revendication 1, dans lequel la largeur, W, va de 240 mm à 600 mm.

3. Procédé selon l'une quelconque des revendications précédentes, dans lequel la densité d'impression maximale de la zone centrale (ZL) est de 0,15.

4. Procédé selon l'une quelconque des revendications précédentes, dans lequel l'étape de découpe des premier et deuxième stratifiés élastiques continus (406, 408) comprend en outre la découpe à travers les première et deuxième zones (ZH1, ZH2).

5. Procédé selon l'une quelconque des revendications précédentes, dans lequel l'étape de découpe des premier et deuxième stratifiés élastiques continus (406, 408) comprend en outre la découpe des premier et deuxième stratifiés élastiques continus (406, 408) en des pièces distinctes ayant une longueur de pas, PL, s'étendant le long du sens machine, la première zone (ZH1) s'étendant en continu dans le sens machine sur une distance inférieure ou égale à 40 % de la longueur de pas, PL.

6. Procédé selon l'une quelconque des revendications précédentes, dans lequel la zone centrale (ZL) du graphisme (G) s'étend en continu dans le sens machine.

7. Procédé selon l'une quelconque des revendications précédentes, dans lequel l'étape de pliage du châssis (102) le long de l'axe latéral (126) comprend en outre l'alignement avec la première zone (ZH1) et la deuxième zone (ZH2) de sorte que les première et deuxième zones forment un dessin contigu sur au moins une couche-culotte (100).

8. Procédé selon l'une quelconque des revendications précédentes, dans lequel le stratifié élastique (402) comprend des première et deuxième régions longitudinales externes (166a, 166b) séparées dans la direction croisée par une région centrale (166c), la région centrale (166c) faisant 33 % de la largeur W du stratifié élastique (402), et la zone centrale (ZL) du graphisme (G) étant positionnée dans la région centrale (166c) du stratifié élastique (402).

9. Procédé selon l'une quelconque des revendications précédentes, dans lequel le graphisme (G) définit un périmètre fermé dans lequel la zone centrale (ZL) comprend une première zone centrale (ZLA) séparée d'une deuxième zone centrale (ZLB) dans le sens machine, les première et deuxième zones (ZH1, ZH2) étant reliées avec et séparées par la première zone centrale (ZLA) et la deuxième zone centrale (ZLB).

10. Procédé selon la revendication 9, dans lequel l'étape de découpe du stratifié élastique (402) le long du sens machine comprend en outre la découpe à travers les première et deuxième zones centrales (ZLA, ZLB).
